# EUROPÄISCHE PATENTANMELDUNG

(11) **EP 2 302 064 A1**
(43) Veröffentlichungstag der Anmeldung: **30.03.2011**
(21) Anmeldenummer: 10181200.6
(22) Anmeldetag: 22.08.2003
(51) Int. Cl.: C12P 7/62, A61K 47/48

(54) **Verfahren zur Herstellung von biologisch abbaubaren, funktionalisierten Polymerpartikeln und deren Verwendung als Arzneimittelträger**

(30) Priorität: 30.08.2002 DE 10240035
(62) Teilanmeldung aus: 03747816.1
(71) Anmelder: MASSEY UNIVERSITY, Palmerston North (NZ)
(72) Erfinder: Rehm, Bernd Helmut Adam, Palmerston North (NZ)
(74) Vertreter: Donald, Jenny Susan

(57) **Zusammenfassung**

Die vorliegende Erfindung betrifft ein Verfahren zur Herstellung von biologisch abbaubaren, funktionalisierten Polymerpartikeln und deren Verwendung ala Arzneimittelträger.

## Beschreibung

Die vorliegende Erfindung betrifft ein Verfahren zur Herstellung von biologisch abbaubaren, funktionalisietten Polymerpartikeln und deren Verwendung als Arzneimittelträger.

### Stand der Technik

Die Effektivität vieler Wirkstoffe hängt davon ab, ob sie genau an den gewünschten Zielort im tierischen Körper transportiert weiden, da die verwendeten Wirkstoffe häufig nur an einer bestimmten Stelle ihre volle Winkung entfalten können und an anderen Stellen nicht wirken oder dort sogar negative Auswirkungen haben Letzteres gilt besonders bei der Bekämpfung von krebsartigen Erkrankungen und insbesondere von krebsartigen Erkrankungen, die im Zentralen Nervensystem (ZNS) auftreten

Ein gutes Beispiel für die Schwierigkeiten, die beim Transport von Wirkstoffen im tierischen Körper auftreten, bietet die Blut-Him-Schranke (BHS). Dort müssen Wirkstoffe, die ihren Zielort im ZNS haben, zuvor diese Barriere überwinden. Dieses Netzwerk von Blutgefäßen und Zellen schützt das ZNS und verhindert, dass nicht wasserlösliche Substanzen in das ZNS eindringen können. Fettlösliche Substanzen können diese Schranke ohne Schwierigkeiten durch einfache Diffusion durchschreiten Für den Transport von polaren Substanzen und Ionen sind allerdings aktive und passive Transportsysteme notwendig. Da aber z.B mehr als 98% aller neu entdeckten Medikamente, deren Zielort im ZNS liegt, nicht wasserlöslich sind, können sie die BHS nicht überqueren und somit ihren Zielort nicht erreichen.

Anhand dieses Beispiels sollen die Schwierigkeiten aufgezeigt werden, die beim Transport von Wirkstoffen im tierischen Organismus auftreten können. Bei der BHS wird zum Beispiel versucht durch Veränderung der Peirmeabilität der Membran, diese für Wirkstoffe durchlässiger zu machen. Die Durchlässigkeit der BHS für Wirkstoffe kann zum Beispiel durch künstliche Erhöhung des osmotischen Drucks oder der Verabreichung von Bradykinin Analoga erreicht werden. Sanovich et al, (Sanovich, E. et al., Brain Res. 1995, Vol.705(1-2), S.125-135) und andere demonstrierten z.B. eine erhöhte Permeabilität der BHS für Lanthan durch gleichzeitige Verabreichung mit dem Bradykinin Analogon RMP-7. Ein grundsätzlicher Nachteil beim Öffnen der BHS durch einen der vorgenannten Mechanismen ist, dass sich die Permeabilität dadurch für alle Substanzen und damit auch für toxische Substanzen, die den Zielzellen im ZNS schaden können, erhöht.

Eine andere Möglichkeit besteht darin, den Witkstoff chemisch so zu modifizieren, dass er lipophiler wird und damit auch leichter die BHS durchschreiten kann, wie es zum Beispiel an Chlorambucil-Detivaten gezeigt worden ist (Greig, N,H et al., Cancer Chemother. Pharmacol. 1990, Vol.25(5), S.320-325).,

Eine Alternative zu beiden vorgenannten Systemen bietet die Verwendung von Nano- bzw. Mikropartikeln. Durch ihre Verwendung müssen weder die Durchlässigkeit der Membran noch der Wirkstoff modifiziert werden Kreuter (Kreuter, J. J. Anat 1996, Vol. 189(3), S.503-505) zeigt auf, dass die dazu verwendeten Polymerpartikel im wesentlichen durch chemische Verfahren, wie Emulsiospolymersation, Grenzflächenpolymerisation, Desolvatisieren, Abdampfen und Lösungsmittelabscheidung hergestellt werden

Gemäß der DE 197 45 950 A1 werden Polymerpartikel aus den verschiedensten Substanzen (Polymere (hier: Polybutylcyanoacrylat), feste oder flüssige Lipide, o/w-Emulsionen, w/o/w-Emulsionen oder Phospholipidvesikel) verwendet, an die Arzneistoffe gebunden werden, um sie ins ZNS zu transportieren.

Ein anderer kritischer Punkt beim Transport von Nano- und Mikropartikeln über eine Membran und insbesondere die Membranen der BHS, ist die Größe der Polymerpartikel Bisherige Forschungsergebnisse zeigen, dass Polymerpatikel bis zu einer Größe von 270 nm die BHS überwinden können (Lockman, P.R, et al., DrugDevelop. Indust. Pharmacy 2002, Vol.28(1), S.1-12)

Daher ist es wichtig, dass die verwendeten Polymerpartikel ein bestimmte Größe aufweisen. Nach Lockmann (Lockman, P.R, et al., Drug Develop Indust. Pharmacy 2002, Vol.28(1), S.1-12) wird die Größe der durch die nach den obigen Verfahren hergestellten Polymerpartikel meistens durch Photonenkorrelationsspektroskopie bestimmt. Diese auf der Brownschen Molekularbewegung beruhende Technik vermisst das Polymerpartikel mit einem Laserstrahl, wobei die Zeitabhängigkeit der Lichtveränderung zur Bestimmung der Partikelgröße herangezogen wird Jedoch ist dieses Analyseverfahren zur nachträglichen Bestimmung der Größe der hergestellten Polymerpartikel sehr zeit-und kostenaufwendig

Daher ist es die Aufgabe der vorliegenden Erfindung, ein schnell einsetzbares und kostengünstiges Transportsystem für biologisch aktive Substanzen zur Verfügung zu stellen, dass einen effektiven und sicheren Transport von Wirkstoffen im tierischen Organismus ermöglicht

Als "biologisch aktive Substanz" im Sinne dieser Erfindung wird jede Substanz bezeichnet, die eine biologische Reaktion des Organismus auslösen kann. Diese Substanzen umfassen sowohl Enzyme und Abzyme, die eine bestimmte Reaktion im Organismus katalysieren, Proteine, wie zum Beispiel Antikörper, die eine indirekte Reaktion des Organismus auf die Anwesenheit dieser Substanz im Organismus hervorrufen als auch anorganische und organische Moleküle, die nicht biologischen Ursprungs sind, das heißt, nicht von einem natürlich vorkommenden Organismus gebildet werden, sondern künstlich hergestellt werden. Zur letzten Gruppe gehören auch die meisten pharmazeutischen Wirkstoffe. Die biologisch aktiven Substanzen können je nach ihrer Art auch dazu geeignet sein, weitere biologisch aktive Substanzen zu binden

Um die oben genannte Aufgabe zu lösen, wird ein Verfahren zur Herstellung von biologisch abbaubaren Polymerpartikeln bereitgestellt Dieses Verfahren umfasst das Einbringen zumindest eines induzierbaren Gens in einen Mikroorganismus, wobei das Gen für ein Protein codiert, das die Größe der Polymerpartikel kontrolliert und das Kultivieren des Mikroorganismus unter Induktion des oben genannten zumindest einen induzierbaren Gens in einem Kulturmedium unter Bedingungen, die zur Herstellung der biologisch abbaubaren Polymerpartikel durch den Mikroorganismus geeignet sind. Durch dieses Verfahren ist es möglich biokompatible, biologisch abbaubare Polymerpartikel herzustellen, die sich für den Transport von biologisch aktiven Substanzen eignen und wobei durch die Steuerung der Größe der sich bildenden Polymerpartikel, diese je nach Bedarf in gewünschter Größe produziert werden können. Durch die kontrollierte Herstellung von Polymerpartikel bestimmter Größe wird die Ausbeute an Polymerpartikeln der gewünschten Größe erhöht, was zur Steigerung der Effektivität des Verfahrens führt und gleichzeitig hilft Kosten zu sparen. AuBerdem könuen durch das erfindungsgemäße Verfahren Polymerpartikel hergestellt werden, die der bereits zuvor genannten Anforderung an die Partikelgröße für den Transport über die BHS entspreche Durch dieses Verfahren können vor allem Polymerpartikel hergestellt weiden, die kleiner sind als die durch Mikroorganismen natürlich hergestellten Polymerpartikel dieser Art. Die durchschnittliche Größe der durch Mikroorganismen natürlich hergestellten Polymerpartikel beträgt 300 bis 500 nm (Wieczorek, R et al, J. Bacteriol. 199 7, Vo1.177(9), S 2425-2435), während das erfindungsgemäße Verfahren es ermöglicht, die Herstellung der Polymerpartikel so zu steuern, dass sie deutlich unter diesem Durchschnittswert liegen.

Die Induktion des die Partikelgröße bestimmenden Gens erfolgt dabei über einen vorgeschalteten induzierbaren Promotor, wie z.B. einen bad-Promotor, den man durch Arabinose induziert. Die Mikroorganismen, die man dafür einsetzt, besitzen kein Gen für die Kontrolle der Größe der Polymerpartikel oder man inaktiviert dieses Gen und ersetzt es durch das im erfindungsgemäßen Verfahren beschriebene zumindest eine induzierbare Gen, das für ein Protein codiert, das die Größe der Polymerpartikel kontrolliert. Dieses Gen wird dabei über einen Vektor in die Zelle eingebracht, der im experimentellen Teil dieser Beschreibung näher beschrieben wird. Dadurch ist es erstmalig möglich, biokompatible, biologisch abbaubare Polymerpartikel definierter Größe in einem mikrobiellen Verfahren herzustellen.

Diese Polymerpartikel werden als cytoplasmatische Einschlüsse in der Zelle abgelagert. Der Kern dieser Polymerpartikel besteht aus Polyhydroxyalkylcarboxylaten insbesondere Polyhydroxyalkanoaten und wird von einer aus Proteinen und Phospholipiden bestehenden Hüllmembran umgeben Die Hüllmembran besteht aus Lipiden und in sie eingebettete Proteine. Die Polyhydroxyalkylcarboxylate, die den Kern dieser Polymerpartikel bilden, haben Schmelzpunkte von 50°C bis 176°C, eine Kristallinität von 30% bis 70% und Zerreisdehnungswerte von 5% bis 300%.

Um dieses vorteilhafte Verfahren auch in Mikroorganismen zur Anwendung zu bringen, die sich für die biotechnologische Züchtung besser eignen (z.B. bestimmte Formen von *E. coli,* die als GRAS Organismen klassifiziert sind) aber die oben genannten Polymerpartikel aufgrund ihrer genetischen Ausstattung nicht bilden können, bringt man neben dem zumindest einem induzierbaren Gen, das für ein Protein codiert, das die Größe der Polymerpartikel kontrolliert, mindestens ein weiteres Gen ein, das für ein bei der Bildung der Polymerpartikel beteiligtes Protein codiert. Denkbar ist jedes Protein, dass den Stoffwechsel, der zur Bildung der Polymerpartikel führt, beeinflussen kann und damit die Zusammensetzung der sich bildenden Polymerpartikel Dabei wählt man das mindestens eine weitere Gen, das für ein bei der Bildung der Polymerpartikel beteiligtes Protein codiert, so aus, das es für eine Thiolase, eine Reduktase oder eine Polymer-Synthase codiert. Unter einer Polymer-Synthase versteht man jedes Protein, das den letzten Schritt zur Bildung eines Polymers katalysieren kann. Neben den in dieser Erfindung beschriebenen Polymer -Synthasen kann die Bildung eines Polymers z.B. auch von einer Lipase übernommen werden. Bevorzugt wählt man das mindestens eine weitere Gen, das für ein bei der Bildung der Polymerpartikel beteiligtes Protein codiert, so aus, dass es für die phaA-Thiolase, die phaB-Ketoacyl-Reduktase oder die phaC-Synthase aus *Ralstonia eutropha* codiert. Durch das Einbringen diester zusätzlichen Gene wird die Zelle dazu in die Lage versetzt, Proteine zu produzieren, die ihr die Bildung der Polymerpartikel ermöglichen. Durch gezieltes auswählen des mindestens einen weiteren Gens, das für ein bei der Bildung der Polymerpartikel beteiligtes Protein codiert, kann auch die spätere Zusammensetzung der Polymerpartikel beeinflusst werden Gene, die für Proteine codieren, die am Stoffwechselweg zur Bildung der Polymerpartikel beteiligt sind, können unterschiedliche Substratspezifität besitzen, unterschiedliche Reaktionsprodukte bilden oder Abzweigungen im Stoffwechselweg blockieren, um so die Substrate und Moleküle, die an der Bildung der Polymerpartikel beteiligt sind, gezielt zu beeinflussen

Bei Mikroorganismen, wie den in Beispiel 4 beschriebenen Mutanten der Gattung *E. coli,* die einen veränderten Fettsäuremetabolismus aufweisen, benötigt man neben dem zumindest einem induzierbaren Gen, das für ein Protein codiert, das die Größe der Polymerpartikel kontrolliert, nur die Polymer Synthase, um die Herstellung der Polymerpartikel zu ermöglichen. Je nach dem welchen Organismus man verwendet, können weiter Gene in die Zelle eingebracht werden, um die Herstellung der Polymerpartikel unter den gegebenen Bedingungen zu ermöglichen. Enthält die Zelle nicht alle für die Bildung der Polymerpartikel erforderlichen Gene, so kann die Herstellung der Polymerpartikel trotzdem erfolgen, wenn man die durch die fehlenden Proteine hergestellten Zwischenprodukte der Zelle über das Nährmedium zuführt. Für die Bildung der Polymerpartikel ist jedoch immer mindestens eine Polymer-Synthase erforderlich.

Durch die Steuerung der Zusammensetzung der Polymerpartikel können ihre Eigenschaften beeinflusst werden. Durch die Beeinflussung ihrer Eigenschaften kann man zum Beispiel die Geschwindigkeit der biologischen Abbaubarkeit der Polymerpartikel beeinflussen Neben der bereits zuvor geschilderten Möglichkeit, durch die gezielte Auswahl der in die Zelle eingebrachten weiteren Gene, die für ein bei der Bildung der Polymerpartikel beteiligtes Protein codieren, ist zur Beeinflussung der Zusammensetzung der sich bildenden Polymerpartikel *in vivo* besonders bevorzugt, das Einbringen von zumindest einem zusätzlichen Gen in die Zelle, das für eine Thiolase und/oder eine Polymer-Synthase codiert Die unterschiedliche Substratspezifität der Thiolasen und der Polymer-Synthasen führt zu untensehiedlichen Zwischen und Endprodukten und damit zu einer anderen Zusammensetzung des gebildeten Polymerkerns des Partikels.

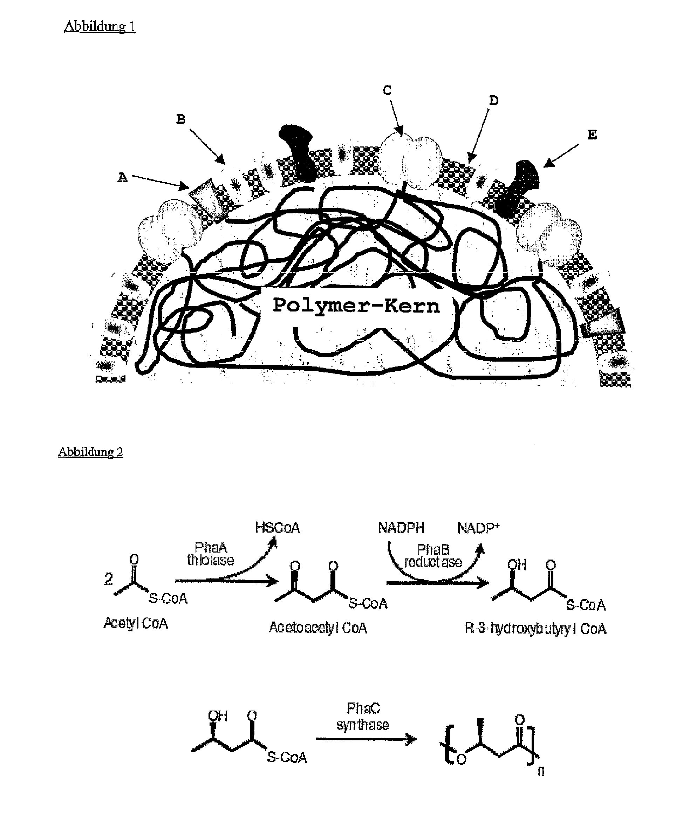

Das Herstellungsprinzip dieser Polymerpartikel wird anhand der Abbildung 2 beispielhaft verdeutlicht. Aktivierte Vorstufen für die Biosynthese der Polymere können grundsätzlich von dem zentralen Metaboliten Acetyl-CoA oder von Intermediaten der primären Stoffwechselwege Citrat-Zyklus, Fettsäure-β-Oxidation und Fettsäure-*de novo*-Biosynthese, sowie dem Stoffwechsel von Aminosäuren abgeleitet werden. Dienen Fettsäuren als Kohlenstoffquelle so werden über die Fettsäure-β-Oxidation Intermediate (Acyl-CoA, insbesondere 3 Hydroxyacyl-CoA) bereitgestellt, die als aktivierte Vorstufen für die PHA-Biosynthese dienen.

Die Kontrolle der Partikelgröße erfolgt, indem man das zumindest eine induzierbare Gen, das für ein Protein codiert, das die Größe der Polymerpartikel kontrolliert, aus der Familie der Phasin ähnlichen Proteine ableitet und bevorzugt aus der Gruppe auswählt, die das Phasin-Gen phaP aus *Ralstonia eutropha* und das Phasin-Gen phaF aus *Pseudomonas oleovorans* umfasst. Phasine sind amphiphile Proteine mit einem Molekulargewicht von 14 bis 28 kDa, die fest an die hydrophobe Oberfläche der Polymerpartikel binden

Polymerpartikel mit unterschiedlicher Zusammensetzung des sie bildenden Polymers weisen unterschiedliche mechanische Eigenschaften auf und setzen biologisch aktive Substanzen, insbesondere pharmazeutische Wirkstoffe, unterschiedlich schnell frei Polymerpartikel zum Beispiel, die aus C6-C14 3-Hydroxyfettsäuren zusammengesetzt sind, zeigen infolge der niedrigen Kirstallinität des Polymers eine höhere Abbaurate des Polymers auf. Für gewöhnlich verringert eine Erhöhung des molaren Verhältnisses der Polymerbestandteile mit größeren Seitenketten am Polymerrückgrat die Kristallinität und führt zu erhöhten elastomeren Eigenschaften. Durch die Kontrolle der Polymerzusammensetzung gemäß dem in der Erfindung beschriebenen Verfahren kann somit die biologische Abbaubarkeit der Polymerpartikel beeinflusst werden und damit auch die Freisetzungsrate für biologisch aktive Substanzen, insbesondere pharmazeutischen Wirkstoffe.

Vorzugsweise bringt man in das Kulturmedium als Substrat für die Bildung der Polymerpartikel mindestens eine Fettsäure mit funktionellen Seitengruppen und besonders bevorzugt mindestens eine Hydroxyfettsäure und/oder mindestens eine Mercaptofettsäure und/oder mindestens eine β-Aminofettsäure ein Unter "Fettsäuren mit funktionellen Seitengruppen" sind gesättigte oder ungesättigte Fettsäuren zu verstehen Darunter fallen auch Fettsäuren, die funktionelle Seitengruppen enthalten, die ausgewählt werden aus der Gruppe, die Methylgruppen, Alkylgruppen, Hydroxylgruppen, Phenylgruppen, Sulfhydrylgruppen, primäre, sekundäre und tertiäre Aminogruppen, Aldehydgruppen, Ketogruppen, Ethergruppen, Carboxylgruppen, O-Estergruppen, Thioestergruppen, Carbonsäureamidgruppen, Halbacetalgruppen, Acetalgruppen, Phosphatmonoestergruppen und Phosphatdiestergruppen umfasst.

Der Einsatz der Substrate richtet sich nach der gewünschten Zusammensetzung und den gewünschten Eigenschaften der Polymerpartikel, die sowohl genetisch durch den Einsatz verschiedener Gene, die für Proteine mit unterschiedlicher Substratspezifität codieren als auch durch die verwendeten Zusatzstoffe, Substrate und Reaktionsbedingungen, die im Kulturmedium vorliegen, beeinflusst werden.

Um eine noch genauere Kontrolle der Größe der sich bildenden Polymerpartikel zu erlangen, gibt man das Substrat dem Kulturmedium in einer solchen Menge zu, dass es ausreichend bemessen ist, um die Kontrolle der Größe der Polymerpartikel zu gewährleisten. Dadurch ist eine zusätzliche Möglichkeit gegeben, die Steuerung der Partikelgröße noch wirksamer zu kontrollierten.

Für die Bildung der Polymerpartikel nach dem erfindungsgemäßen Verfahren wählt man den verwendeten Mikroorganismus aus der Gattung aus, die *Ralstonia, Alcaligenes, Pseudomonas* und *Halobiforma* umfasst. Bevorzugt wählt man den verwendeten Mikroorganismus aus der Gruppe aus, die *Ralstonia eutropha, Alcaligenes latus, Escherichia coli, Pseudomonas fragi, Pseudomonas putida, Pseudomonas oleovorans, Pseudomonas aeruginosa, Pseudomonas fluorescens,* und *Halobiforma haloterrestris* umfasst. Diese Gruppe umfasst sowohl Mikroorganismen, die von Natur aus in der Lage sind, biokompatible, biologisch abbaubare Polymerpartikel herzustellen, als auch Mikroorganismen, wie zum Beispiel *E. coli,* die dazu aufgrund ihrer genetischen Ausstattung nicht in der Lage sind In die zuletzt genannten Mikroorganismen bringt man die dafür erforderlichen Gene nach dem erfindungsgemäßen Verfahren ein, um sie zur Herstellung der Polymerpaitikel zu befähigen. Prinzipiell kann man mit dem oben beschriebenen Verfahren jeden kultivierbaren Mikroorganismus für die Herstellung von Polymerpartikel verwenden, auch wenn er die für die Polymerpartikelbildung erforderlichen Substrate aufgrund eines anderen Stoffwechsels nicht herstellen kann. In solchen Fällen gibt man die erforderlichen Substrat dem Kulturmedium zu, die dann von den Proteinen, die von den Genen explimiert worden sind, die in die Zelle eingebracht worden sind, zu Polymerpartikeln umgesetzt wenden.

Um die Polymerpartikel aus den Zellen zu erhalten, schließt man die kultivierten Mikroorganismen auf an sich bekannte Weise auf und trennt die Polymerpartikel anschließend von den Zellresten ab. Um den Größenbereich der so gewonnenen Polymerpartikel noch weiter einzuengen, können Standardverfahren, wie z B eine Ausschlusscbromatographie oder eine Dichtegradientenzentrifugation, zur Selektion der Polymerpartikel der gewünschten Größe eingesetzt werden.

Die aus Proteinen und Lipiden bestehende Hüllmembran der eifindungsgemäß hergestellten Polymerpartikel kann man ebenfalls modifizieren, um den Partikeln günstigere Eigenschaften für den Transport der Wirkstoffe im tierischen Körper zu verleihen. Dafür trennt man eine sich auf der Oberfläche der Polymerpartikel befindliche Lipidschicht von den gemäß dem erfindungsgemäßen Verfahren erhaltenen Polymerpartikeln ab, und ersetzt sie durch eine Lipidschicht anderer Zusammensetzung

Beim Ersetzen der Lipidschicht durch eine Lipidschicht anderer Zusammensetzung sind die Eigenschaften der neuen Lipidschicht von Bedeutung, die den Transport der Polymerpartikel über eine biologische Membran beeinflussen Wenn die Lipidschicht der Hüllmembran mit der Lipidschicht der Ziehnembran übeneinstimmt, kann eine bessere Aufnahme von Partikeln beobachtet werden (Femart, L. et al., J Pharmacol Exp. Ther. 1999, Vol.291(3), S.1017-1022), Deshalb kann die Lipidschicht der Polymerpartikel entfernt werden und durch eine Lipidschicht anderer Zusammensetzung ersetzt werden.. Dies geschieht vorzugsweise durch eine Aceton-Extraktion oder es werden Phospholipasen oder nichtdenaturierende Detergentien verwendet Anschließend wird eine Mischung der entsprechenden amphiphilen Moleküle zu den nun nahezu Lipid-freien Polymerpartikeln gegeben, um eine Lipidschicht gewünschter Zusammensetzung zu erhalten. Die neue Lipidschicht besteht dabei bevorzugt aus einer Mischung von amphiphilen Molekülen, die man aus der Gruppe auswählt, die Phospholipide und Etherlipide umfasst.

Neben den Lipiden kann man durch den Einsatz der oben genannten Detergenzien auch die sich auf der Oberfläche der Polymerpartikel befindlichen Proteine, bis auf die Polymer-Synthase, entfernen und durch funktionalisierte Proteine ersetzen. Dadurch kann man nackte Polymerpartikel für eine Vielzahl von Modifikationsmöglichkeiten bereitstellen, die im Weiteren noch beschrieben werden Für diese Art der Modifikation eignen sich besonders Polymerpartikel, die aus dem Mikroorganismus *H. haloterrestris* gewonnen werden

Je nach späterer Anwendung der Polymerpartikel steuert man die Partikelgröße durch das zumindest eine induzierbare Gen so, dass die gebildeten Polymerpartikel einen Durchmesser von 10 nm bis 3 µm, bevorzugt 1 nm bis 900 nm und besonders bevorzugt 10 nm bis 100 nm haben. In einer besonderen Ausfühtungsform der vorliegenden Erfindung kann man diese Größenkontrolle auch durch die Steuerung der Verfügbarkeit eines Substrates im Kulturmedium oder durch eine Kombination beider Mechanismen erzielen

Für die Funktionalisierung der nach dem erfindungsgemäßen Verfahren hergestellten Polymerpartikel ist es erforderlich, dass das im Verfahren eingebrachte zumindest eine induzierbare Gen, das für ein Protein codiert, das die Größe der Polymerpartikel kontrolliert, eine Polymerpaitikelbindedomäne und zumindest eine Bindedomäne umfasst, wobei die zumindest eine Bindedomäne eine biologisch aktive Substanz und/oder ein Kopplungsreagenz binden kann, Besonders bevorzugt ist ebenfalls, dass das mindestens ein weitere Gen, das für ein bei der Bildung der Polymerpartikel beteiligtes Protein codiert, eine Polymerpartikelbindedomäne und zumindest eine Bindedomäne umfasst, wobei die zumindest eine Bindedomäne eine biologisch aktive Substanz und/oder ein Kopplungsreagenz binden kann.

Eine "Bindedomäne" bezeichnet den Teil des Proteins, den man durch genetische Modifikation des zuvor in die Zelle eingebrachten zumindest einen induzierbaren Gens und/oder des mindestens einen weiteren Gens, das für ein bei der Bildung der Polymerpartikel beteiligtes Protein codiert, erhält, Die Bindedomäne, die biologisch aktive Substanzen und/oder ein Kopplungsreagenz binden kann, wählt man aus der Gruppe aus, die Oligopeptide, Enzyme, Abzyme und nicht-katalytische Proteine umfasst, Bevorzugt umfasst diese Gruppe FLAG-Epitope oder mindestens ein Cystein. Diese Gruppen werden im experimentellen Teil auch als Funktionsproteine bezeichnet Die codierende Sequenz für die in dieser Gruppe genannten Mitglieder wird in die codierende Sequenz des zuvor in die Zelle eingebrachten zumindest einen induzierbaren Gens und/oder des mindestens einen weiteren Gens, das für ein bei der Bildung der Polymerpartikel beteiligtes Protein codiert, eingefügt Dadurch werden Proteine produziert, die nicht nur an der Bildung oder der Kontrolle der Bildung der Polymerpartikel beteiligt sind, sondern auch noch über ihre durch genetische Modifikation erhaltene Bindedomäne biologisch aktive Substanzen und/oder Kopplungsreagenzien binden können. Die Bindedomäne kann auch noch nach Herstellung der Polymerpartikel durch chemische Modifikation der auf der Oberfläche lokalisierten Proteine mit Kopplungsreagenzien erhalten wenden (siehe Beispiel 8)

Ein "Kopplungsreagenz" in diesem Sinne ist eine anorganische oder organische Verbindung, die dazu geeignet ist, auf der einen Seite eine biologisch aktive Substanz oder weitere Kopplungsreagenzien und auf der anderen Seite die Bindedomäne an sich zu binden

Dieser Aufbau ermöglicht es, multifunktionelle Polymerpartikel herzustellen, die für den Transport von biologisch aktiven Substanzen geeignet sind Die "Polymerpartikelbindedomäne" besteht bevorzugt aus einem Teil eines Proteins, der es dazu befähigt, auf der hydrophoben Oberfläche der Polymerpartikel anzubinden, Die Polymerpartikeldomäne, die einen Teil eines auf der Oberfläche des Polymerpartikels gebundenen Proteins umfasst, wählt man dabei aus der Gruppe von Proteinen aus, die eine Polymer" Depolymerase, einen Polymer-Regulator, eine Polymer-Synthase und ein die Partikelgröße kontrollierendes Proteine umfässt Diese Proteine stammen bevorzugt aus Mikroorganismen, die in der Lage sind Polymerpartikel zu bilden, insbesondere solche aus der Gattung *Ralstonia, Alcaligenes* und *Pseudomonas* Das die Partikelgröße kontrollierende Protein wird dabei bevorzugt aus der Familie der Phasin-ähnlichen Proteine abgeleitet und noch bevorzugter verwendet man dafür das Phasin aus *R. eutropha* und *P. oleovorans*

Ein "Polymer-Regulator" im Sinne der Erfindung ist ein Protein, welches die Transkription der an der Bildung der Polymerpartikel beteiligten Gene phaA, phaB und phaC reguliert Es wird durch Bindung an die Partikeloberfläche der Tianslaiptionsregalation entzogen, Ein Beispiel eines solchen Regulators ist der Phasin-Repressor (phaR) aus *R eutropha,* der an den Promotor eines Phasin-ähnlichen Gens bindet, dessen Expressionsprodukt die Größe der sich bildenden Polymerpartikel reguliert und verhindert, dass das Gen abgelesen wird. Durch das Binden des Phasin-Repressors an der Oberfläche der sich bildenden Polymerpartikel wird diese Stelle auf dem Promotor freigegeben und die Transkription des dahinterliegenden Gens kann beginnen

Die Nutzung der Bindedomäne einer Polymer-Synthase zur Bindung von Kopplungsreagenzien und/oder biologisch aktiven Substanzen, ergibt sich aus der hohen Stabilität der Bindung zwischen der Polymerpartikelbindedomäne dieses Proteins und dem Kern des Polymeipartikels, Der Erfinder hat überraschend herausgefunden, dass diese Bindung weder durch denaturierenden Reagenzien, wie z.B. Sodiumdodecylsulfat (SDS), Harnstoff, Guanidiumhydrochlorid oder Dithiothreitol, noch durch Verwendung azider Bedingungen vom Kern des biologisch abbaubaren Polymerpartikels gelöst werden kann Bevorzugt wird dafür die Polymer-Synthase verwendet, die aus *R. eutropha, P oleovorans, P. putida* oder *P. aeruginosa* abgeleitet wird,

Dabei ist es ein besonderer Vorteil des erfindungsgemäßen Verfahrens, dass die gentechnische Modifikation der auf der Oberfläche der Polymerpartikel bindenden Proteine nicht die Funktionalität des an der Bildung der Polymerpartikel beteiligten Proteine beeinflusst. So bleibt zum Beispiel die Funktionalität der Polymer-Synthase erhalten, wenn mit ihrem N-terminalen Ende ein Protein fusioniert wird und somit eine Bindedomäne zur Bindung von biologisch aktiven Substanzen und/oder Kopplungsreagenzien erzeugt wird Sollte die Funktionalität eines Proteins durch die Fusion doch beeintnächtigt sein, kann durch die Anwesenheit eines weiteren Gens, das für ein Protein codiert, dass die gleiche Funktion ausführt und in einem aktiven Zustand vorliegt, dieser Mangel kompensiert werden,

Dadurch kann eine stabile Verbindung der über die Bindedomäne der Proteine, insbesondere der Polymer-Synthase, gebundenen biologisch aktiven Substanzen und/oder Kopplungsreagenzien an das Polymerpartikel gewährleistet werden.

Bei der gentechnischen Modifikation der Gene, die für Proteine codieren, die nach ihrer Expression an die Partikeloberfläche binden, können auch Gene mit unterschiedlichen Modifikationen in die Zelle eingebracht werden, Nach der Expression dieser Proteine mit ihren unterschiedlichen Bindedomänen und der Bildung der Polymerpartikel kann so eine Multifunktionalisierung der Partikeloberfläche über die unterschiedlichen Bindedomänen erfolgen Mit Hilfe dieses Verfahrens wird eine einfache und effiziente Massenproduktion von funktionalisierten Polymerpaitikeln ermöglicht

Für die nachträgliche Funktionalisienmg der auf der Oberfläche der Polymerpartikel gebundenen Proteine verwendet man Kopplungsreagenzien, die man bevorzugt aus der Gruppe auswählt, die Bis-(2-oxo-3-oxazolydmyl)-Phosphonchlorid (BOP-C1), Bromo-tris-pyrrolidino-phosphoniumhexa-fluorophosphat (PyBroP), Benzotriazole-1-yl-oxy-tris-pyrrolidino-pbosphoniumhexafluolophosphat (PyBOP), N-HydioxysuccinimidBiotin, 2-(1H-benzotriazole-1-yl)-1,1,3,3-tetramethyluroniumhexafluorophosphat (HBTU), Dicyclohexyl-carbodiimide, Disuccinimidylcarbonat, 1-(3-dimethylaminopropyl)-3-ethylcarbodiimid (EDC), Bis-(2-oxo-3-oxazolydinyl)-phosphin, Diisopropylcarbodiimide (DIPC), 2-(1H-benzotrioxazolyl)-1,1,3,3-tetramethyluroniumtetrafluoroborat (TBTU), 2-(5-norboren-2,3-dicarboxyimido)-1,1,3,3-tetramethyluronumterafluoroborat (TNTU), para-Nirophenylchloroformate, und O-(N-succinimidy)-1,1,3,3- tehramethyluronium tetrafluoroborate (TSTU) umfasst

Als biologisch aktiven Substanzen verwender man Bevorzugt, Pestizide, Herbizide, pharmazeutisch aktive Substanzen und Proteine.

Die pharmazeutisch aktiven Substanzen wählt man aus der Gruppe aus, die Dideoxymosin, Floxuridin, 6-Mercaptopurin, Doxorubicin, Daunozubicin, 1-Darubicin Cisplatin, Methotrexat, Taxol, Antibiotika, Anticoaguranzien, Germizide, Antiarrythmische Agenzien und Wirkstoffvorstufen und -derivate der au geführten Wirkstoffgruppen umfasst.

Die Proteine wählt man bevorzugt aus der Gruppe aus, die Insulin, Calcitonin, ACTH, Glucagon, Somatostatin, Somatotropin, Somatomedin, Parathyroidhormon, Erythropoietin, hypothalamische Freisetzungfaktoren, Prolactin, Thyroid-stimulierendes Hormon, Endorphine, Enkephaline, Vasopressine, nicht natürlich vorkommende Opiate, Superoxiddismutase, Antikörper, Interferone, Asparaginase, Arginase, Arginindeaminase, Adenosindeaminase, Ribonuclease, Trypsin, Chymotrypsin und Pepsin umfasst

Ein weiterer Aspekt der Erfindung ist ein Verfahren zur *in vitro* Herstellung von biologisch abbaubaren Polymerpartikeln, wobei das Verfahren das Bereitstellen einer für die Polymerpartikelbildung geeigneten Lösung mit zumindest einem Substrat, das Einbringen eines Proteins in die Lösung, das dazu geeignet ist, die Größe der Polymerpartikel zu kontrollieren und das Einbringen mindestens eines weiteren Proteins, das an der Bildung der Polymerpartikel beteiligt ist, umfasst. Auch das *in vitro* Verfahren bietet den Vorteil, das die Größe der Polymerpartikel schon während der Herstellung der Polymerpartikel gesteuert werden kann und eine spätere kostenaufwendige und zeitraubende Größenbestimmung und Auftrennung der gebildeten Polymerpartikel in einzelne Größenklassen vermieden wird.

Dabei setzt man für das mindestens eine weitere Protein, das an der Bildung der Polymerpartikel beteiligt ist, eine Polymer-Synthase ein, wobei man diese Polymer-Synthase bevorzugt aus der Gruppe auswählt, welche die Polymer-Synthase aus *R. eutropha, P. oleovorans, P putida* und *P. aeruginosa* umfasst.

Im Gegensatz zur *in vivo* Synthese ist eine *in vitro* Synthese, bei der im Labor aus Mikroorganismen isolierten Proteine und Enzyme verwendet werden, normalerweise sehr kostenintensiv, da sowohl die Enzyme und teilweise auch die Enyzmsubstrate zuvor isoliert und gereinigt werden müssen In einer besonderen Ausfübrungsform der vorliegenden Erfindung zur *in vitro* Synthese der biokompatiblen, biologisch abbaubaren Polymerpartikel gibt man der für die Polymerpartikelbildung geeigneten Lösung mindestens eine Fettsäure, besonders bevorzugt eine β-Mercaptosäure und/oder eine β-Aminofettsäure und eine Acyl-CoA Oxidase oder andere oxidierende und aktivierende Enzyme für die Bildung der Polymerpartikel zu. Durch die Verwendung dieser Substrate anstelle der R/S-3-Hydroxyfettsäuren und der Acyl-CoA Synthetase entsteht ein CoA-Wiedeiverweitungssystem, in dem die Acyl-CoA Oxidase die Fettsäure aktivieren und oxidieren wird, während sie CoA verbraucht und ATP hydrolysiert Während der Polymerisierung spaltet die Polymer- Synthase CoA ab, das dann wiederum von der Acyl-CoA Oxidase verwendet werden kann. Die Kosten dieses *in vitro* Verfahrens lassen sich dadurch merklich verringern.

Ein weiterer Vorteil des *in vitro* Verfahrens zur Herstellung der Polymerpartikel ist, dass man das zumindest eine Substrat der für die Polymerpartikelbildung geeigneten Lösung in einer solchen Menge zugibt, dass es ausreichend bemessen ist, um die Kontrolle der Größe der Polymerpartikel zu gewährleisten.

Außerdem kann die Größe der sich bildenden Polymerpartikel auch noch dadurch kontrolliert werden, indem man die Polymer-Synthase der Lösung in einer solchen Menge zugibt, die ausreichend ist, um die Kontrolle der Größe des sich bildenden Polymerpartikel zu gewährleisten. Wie beim *in vivo* Verfahren gibt es also auch im *in vitro* Verfahren weitere Möglichkeiten, die Steuerung der Polymerpartikelgröße noch genauer zu kontrollieren und damit die Ausbeute an Polymerpartikeln der gewünschten Größe zu erhöhen und damit das Verfahren effizienter und kostengünstiger zu gestalten

Die eigentliche Kontrolle der Partikelgröße erfolgt beim *in vitro* Verfahren allerdings durch Eindringen eines die Größe der Polymerpartikel kontrollierenden Proteins in die Lösung, das man aus der Familie der Phasin-ähnlichen Proteine ableitet und bevorzugt aus der Gruppe auswählt, die das Phasin aus *Ralstonia eutropha* und das Phasin aus *Pseudomonas oleovorans* umfasst.

Durch die Wahl des zumindest einen Substrates als auch der verwendeten Enzyme ist es, wie beim *in vivo* Verfahren auch, möglich, die Zusammensetzung des Polymers zu regulieren und dadurch Polymerkerne mit unterschiedlichen Eigenschaften zu gewinnen Bei Verwendung von mehr als einem Substrat, können zum Beispiel je nach Art der aus den Enzymen gebildeten Polymeren, Polymerpartikel mit unterschiedlicher Zusammensetzung des Polymerkerns gewonnen werden. Wie bereits weiter oben beschrieben verleihen die dadurch hergestellten Polymere dem Polymerpartikel unterschiedlichste Eigenschaften.

Im *in vitro* Verfahren zur Herstellung der Polymerpartikel gibt man zur Kontrolle der Zusammensetzung der Lipidschicht auf der Oberfläche des Polymerpartikels mindestens ein amphiphiles Molekül aus der Gruppe der Phospholipide und Etherlipide zur Lösung hinzu (Ohne zusätzliche amphiphile Moleküle enstehen in vitro Partikel, die nur von Proteinen umgeben sind, was einen weiteren Partikeltyp darstellt.). So lassen sich zum Beispiel Polymerpartikel mit einer spezifischer Oberflächenladung herstellen, die speziell auf die später zu überwindende biologische Membran im tierischen Körper abgestimmt ist. Der Vorteil bei der Modifikation der Lipidschicht der Hüllmembran im *in vitro* Verfahren ist, dass die nachträgliche Modifikation der Lipidschicht, wie beim *in vivo* Verfahren, entfällt. Da die amphiphilen Moleküle, wie zum Beispiel Phospholipide oder Etherlipide, bereit zur Ausgangslösung zugegeben werden, wird von Anfang an eine Lipidschicht mit gewünschter Zusammensetzung erzeugt.

Ein weiterer Aspekt des *in vitro* Verfahrens, der auch im *in vivo* Verfahren zur Anwendung kommt, ist, dass man der für die Polymerpartikelbildung geeigneten Lösung mindestens eine pharmazeutisch aktive Substanz zugibt. Diese wird während der Polymerpartikelsynthese in das Polymerpartikel eingebaut und kann später im tierischen Körper über Diffusion durch die Partikelmatrix oder durch Abbau des Polymerpartikels freigesetzt werden Letztgenannte Variante hat bei den durch das erfindungsgemäße Verfahren hergestellten Polymerpartikeln den weiteren Vorteil, dass die Geschwindigkeit des biologischen Abbaus der Polymerpartikel durch die oben beschriebene Steuerung der Zusammensetzung des Polymerkerns reguliert werden kann. Dadurch ist eine kontinuierliche Freisetzung des Wirkstoffes über einen bestimmten Zeitraum möglich.

Neben dem Einbau von Wirkstoffen in das wachsende Polymerpartikel, erfolgt die Funktionalisierung, wie beim *in vivo* Verfahren, dadurch, dass man mindestens eines der in die für die Polymerpartikelbildung geeigneten Lösung eingebrachten Proteine so auswählt, dass das mindestens eine eingebrachte Protein eine Polymerpartikelbindedomäne und zumindest eine Bindedomäne umfasst, wobei die zumindest eine Bindedomäne eine biologisch aktive Substanz und/oder ein Kopplungsreagenz binden kann. Die hier verwendeten Proteine für die Bildung der Polymerpartikel im *in vitro* Verfahren kann man aus dem weiter oben beschriebenen *in vivo* Verfahren gewinnen und sie weisen dann, je nach Art ihrer oben beschriebenen Herstellungsart, die entsprechenden Eigenschaften auf. Sie werden dann in die für die Polymerpartikelbildung geeigneten Lösung gegeben und sind dort an der Bildung der Polymerpartikel beteiligt. Wie im *in vivo* Verfahren kann man die so hergestellten Polymerpartikel auch nach ihrer Herstellung noch durch nachträgliche Modifikation mit den bereits weiter oben beschriebenen Kopplungsreagenzien modifizieren oder durch Zugabe von biologisch aktiven Substanzen, die an die Bindedomäne der Proteine binden, die auf der Oberfläche der Polymerpartikel gebunden worden sind. Die Erfindung umfasst des weiteren ein Polymerpartikel mit definierter Größe, mit einer Oberflächenschicht aus amphiphilen Molekülen, in die mindestens ein Protein, das aus der Gruppe ausgewählt wird, die eine Polymer Depolymerase, einen Polymer-Regulator, eine Polymer-Synthase und ein die Partikelgröße beeinflussendes Protein umfasst, wobei das mindestens eine Protein eine Polymerpartikelbindedomäne und eine Bindedomäne, die eine biologisch aktive Substanz und/oder ein Kopplungsreagenz binden kann, umfasst und das in einer bevorzugten Ausführungsform nach dem oben beschriebenen Verfahren hergestellt wird.

Aufgrund seiner vorteilhaften Eigenschaften eignen sich die Polymerpartikel der vorliegenden Erfindung besonders für die Herstellung eines Arzneimittels, eines Pestizids oder eines Herbizids, wobei das Arzneimittel bevorzugt für die Behandlung von Erkrankungen des zentralen Nervensystems geeignet ist. Durch die durch das vorliegende Verfahren beschriebenen Modifikationsmöglichkeiten der Polymerpartikel werden die Voraussetzungen für die Überwindung der BHS gegeben

Die Kontrolle der Partikelgröße, die Steuerung der Zusammensetzung der Hüllmembran und insbesondere auch die Funktionalisierung der Partikeloberfläche, machen diese biologisch abbaubaren Polymerpartikel zu einem geeigneten Transportvehikel für biologisch aktive Substanzen aller Art und ermöglichen außerdem auch den zielgerichteten Transport der Polymerpartikel an ihren Wirkort. Die Multifunktionalisierung erlaubt zum Beispiel sowohl das gleichzeitige Anbinden von mindestens einer pharmazeutischen aktiven Substanz an die Partikeloberfläche als auch eines Antikörpers, der aufgrund seiner Bindungsspezifität eine genaue Ansteuerung des Zielortes ermöglicht Diese und weitere Vorteile werden anhand der folgenden Ausführungsbeispiele näher dargestellt.

### Beschreibung der Figuren

Abbildung 1 stellt eine schematische Übersicht eines *in vivo* hergestellten biologisch abbaubaren Polymerpartikels und der Proteine und Lipide dar, die an die Oberfläche gebunden sind.

Dabei beziehen sich die Abkürzungen auf die folgenden Proteine:
**A:** Polymer-Depolymerase
**B:** Phasin (Name des codierenden Gens bei *R. eutropha*: phaP, bei *P. oleovorans*: phaF)
**C:** Polymer-Synthase
**D:** Phospholipid
**E:** Polymer Regulatoren (z.B der Phasin-Repressor (PhaR aus R. eutropha)

Abbildung 2 stellt exemplarisch die Synthese eines der möglichen biologisch abbaubaren Polymere in *R. eutropha* dar Das einfach Polyhydroxyalkanoat Polyhydroxybuttersäure (PHB) wird ausgehend vom Substrat Acetyl-CoA in einem dreistufigen Prozess hergestellt Die sich wiederholende C4-Einheit in PHB ist die β-Hydroxybuttersäure. Der letzte Schritt der Synthese führt zur Bildung des Polymeipaitikels, auf deren Oberfläche die Polymer-Synthase gebunden ist.

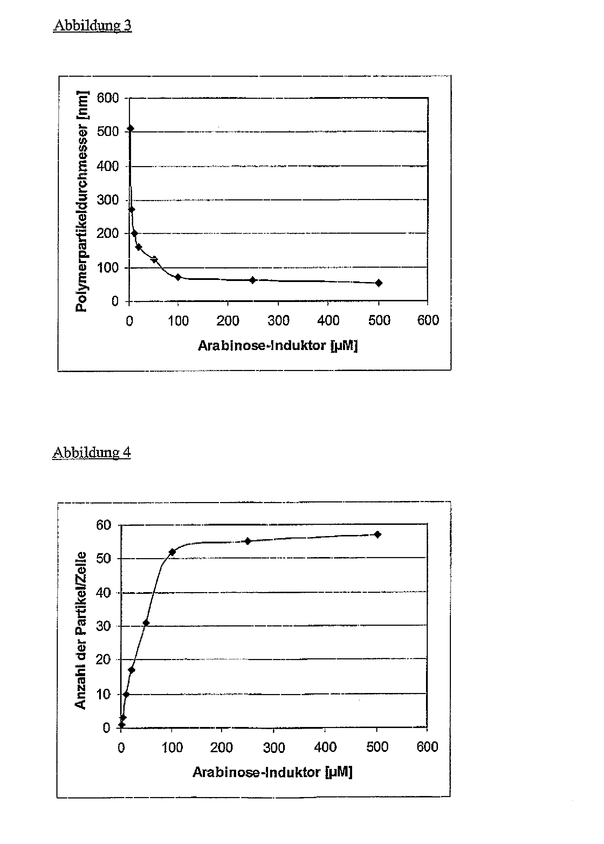

Abbildung 3 und 4 stellen die Ergebnisse aus den *in vivo* Experhnenten dar Abbildung 3 beschreibt dabei das Verhalten des Polymerpartikelduichmessers bei verstärkter Phasinexpression (Erhöhung der Induktozmenge in der Lösung), während Abbildung 4 das Verhalten der Polymeipaxtikelanzahl in der Zelle bei verstärkter Phasinexpression (Erhöhung der Indukiormenge in der Lösung) darstellt.

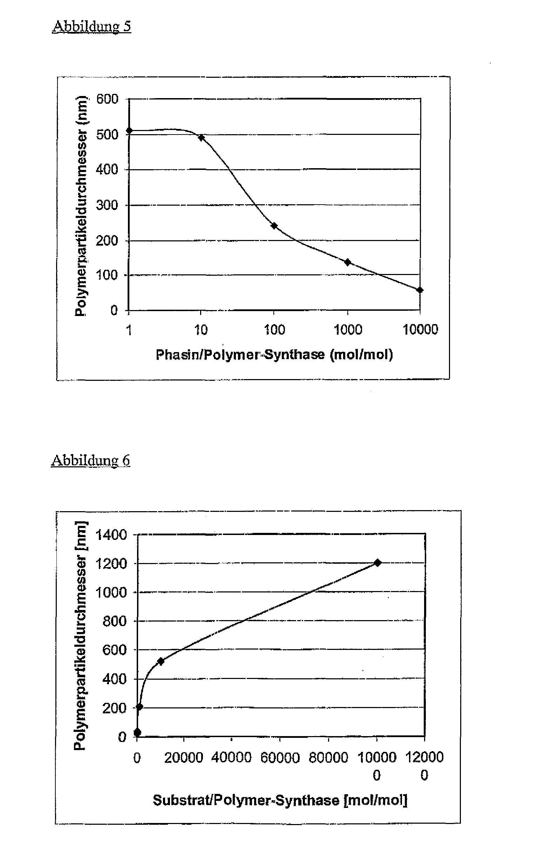

Abbildung 5 und 6 stellen die Ergebnisse der *in vitro* Experimente dar, Abbildung 5 beschreibt den Einfluß des Mengenverhältnisses von Phasin zur Polymer-Synthase auf den Polymerpartikeldurchmesser, während in Abbildung 6 der Einfluss des Verhältnisses von Substrat zur Polymer-Synthase auf den Polymerpartikeldurchmesser dargestellt wird.

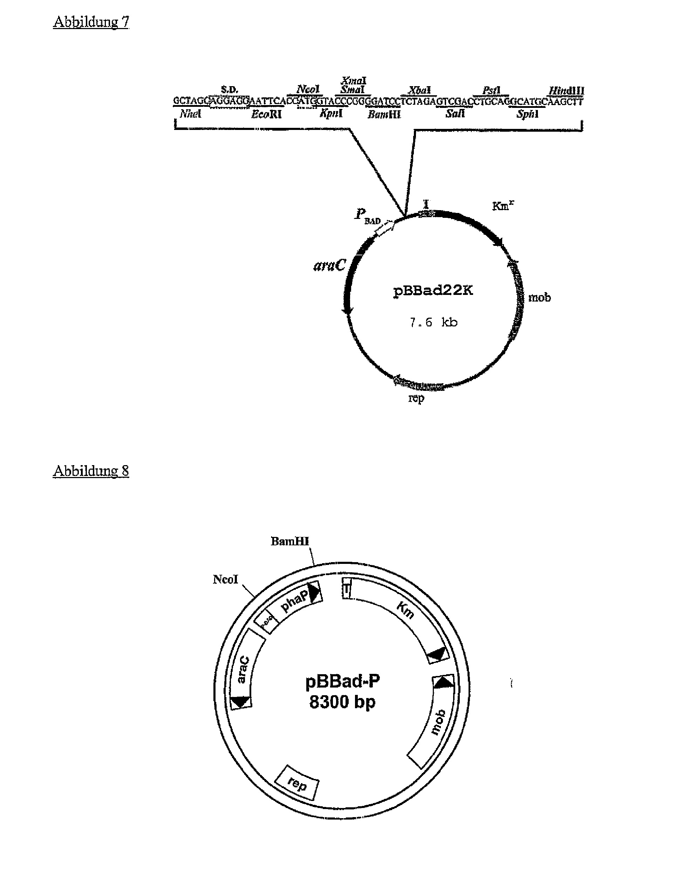

Abbildung 7 stellt den Vektor pBBad22K (Sukchawalit, R. et a1., FEMS Microbiol Lett. 1999, Vol.181(2), S.217-223) dar, der als Ausgangsplasmid zur Konstruktion der Plasmide pBBad-P (trägt das Gen phaP aus *R eutropha*) und pBBad-F (trägt das Gen phaF aus *P. oleovorans*) verwendet wird

Abbildung 8 stellt den Vektor pBBad-P dar, der das Gen phaP für die Expression des die Partikelgrößebestimmenden Proteins Phasin aus *R. eutropha* trägt

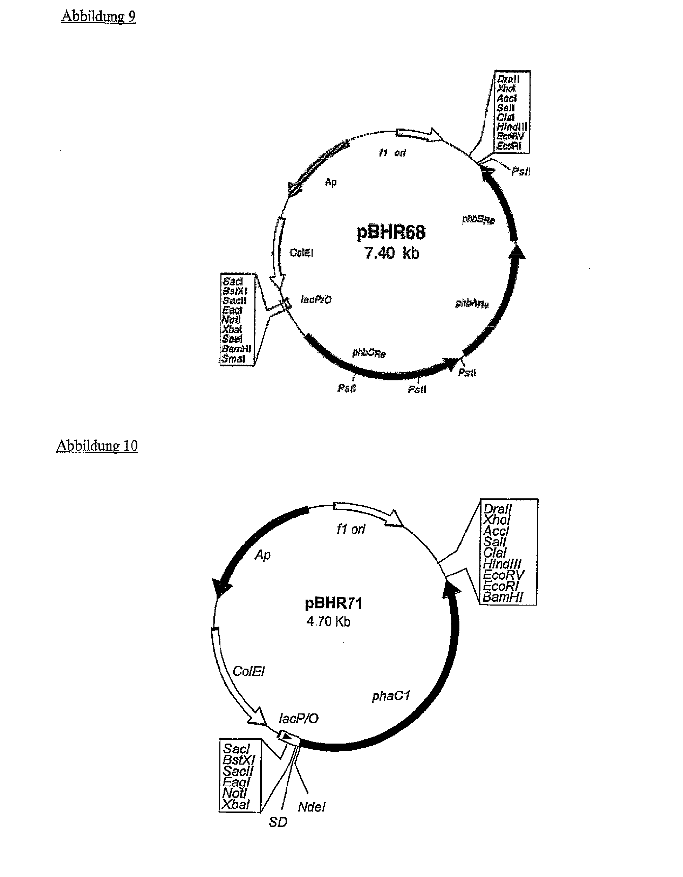

Abbildung 9 stellt das die Polymer(Polyhydroxybuttersäure)-Speicherung vermittelnde Plasmid pBHR68 (Spiekermann, P. et al., Arch. Microbiol. 1999, Vol.171, S.73-80) dar, dass die Gene phB_{Re,} phbA_{Re} und phbC_{Re} aus Ralstonia eutropha trägt, die das Biosyntheseoperon für die Expression der phaA-Thiolase, phaB-Ketoacyl-Reduktase und der phaC-Synthase bilden.

Abbildung 10 stellt das die Polymer(Polyhydroxyallcanoate)-Speicherung vermittelnde Plasmid pBHR71 (Lazlgenbach, S. et al , FEMS Miciobiol. Leet. 1997, Vol.150, S. 303-309) dar, welches das Gen phaC1 für die Expression der Polymer-Synthase trägt.

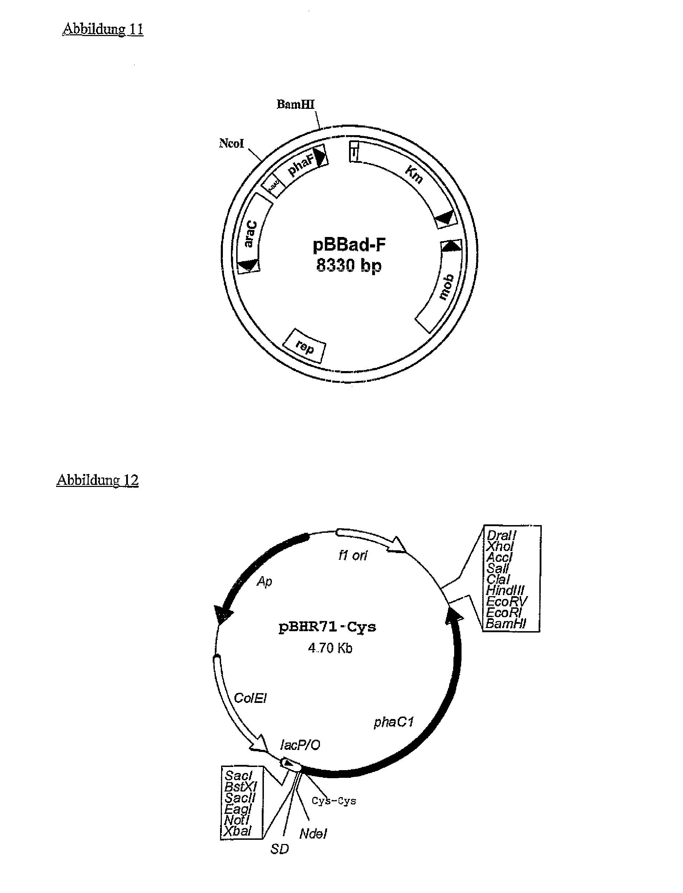

Abbildung 11 stellt den Vektor pBBad-F dar, der das Gen phaF für die Expression des die Partikelgrößebestimmenden Phasin-ähnlichen Proteins aus *P. oleovorans* trägt.

Abbildung 12 stellt den Vektor pBHR71-Cys dar, der ausgehend von dem Plasmid pBHR71 aus Abbildung 10 konstruiert worden ist Das Gen phaC1, dass für die Polymer-Synthase codiert, trägt in dieser Konstruktion zwei Cystein-Reste am N-Teiminus, um die direkte Anbindung von biologisch aktiver Substanzen oder die Anbindung von biologisch aktiven Substanzen über Kopplungsreagenzien, zu ermöglichen.

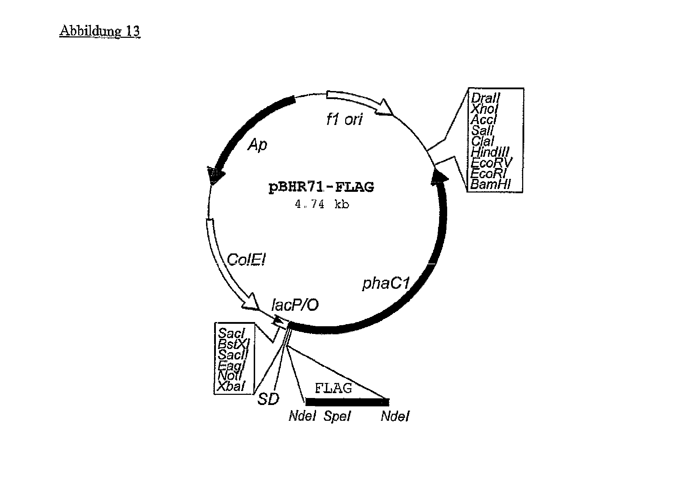

Abbildung 13 stellte den Vektor pBHR71-FLAG dar, der ebenfalls ausgehend von dem Plasmid pBHR71 aus Abbildung 10 konstruiert worden ist. Das Gen phaC1, dass für die Polymer-Synthase codiert, trägt in dieser Konstruktion zwei FLAG-Epitope am N-Terminus. Dies ermöglicht sowohl *in vivo* den Einbau von Gensequenzen für Funktionsproteine über die SpeI-Schnittstelle innerhalb der FLAG Gensequenz als auch die Anbindung von Kopplungsreagenzien und/oder biologisch aktiven Substanzen *in vitro* an die bereits exprimierte Polymer-Synthase. Die verwendeten biologisch aktiven Substanzen, die direkt oder über Kopplungsreagenzien gebunden sind, vermitteln die Bindung am Zielort (z B Zelloberfläche) oder eine biologische Aktivität, insbesondere eine enzymatische Aktivität

### Beispiel 1: Kontrolle der Größe der in vivo hergestellten biologisch abbaubaren Polymerpartikel

### Beispiel 1.1: Herstellung von Polymerpartikeln in R. eutropha

Für die Herstellung der biologisch abbaubaren Polymerpartikel wird eine "*knock-out*" Mutante von *Ralstonia eutropha* (früher *Alcaligenes eutrophus*) York et al. (York, G.M. et al. J. Bacteriol. 2001, Vol 183, S 4217-4226) verwendet, die bezüglich des Gens, dass für die Expression des Oberflächenproteins auf den Polymerpartikeln Phasin codiert, einen defekt aufweist (phaP(-)), wodurch der Onganismus nicht mehr in der Lage ist, dass durch das phaP-Gen codierte Phasin zu exprimieren Des Weiteren können zur Herstellung der biologisch abbaubaren Polymerpartikel auch Mikroorganismen eingesetzt werden, welche dieses Gen und die weiteren für die Biosynthese von Polymerpartikeln erforderlichen Gene nicht enthalten. Beispiele für solche Mikroorganismen wären *Escherichia coli,* und *Halobiforma haloterrestris.* In einem Ausführungsbeispiel wird *Escherichia coli* (siehe Beispiel 2), das naturgemäß nicht in der Lage ist, die bereits zuvor beschriebenen biologisch abbaubaren Polymerpartikel herzustellen, verwendet.

Die zuletzt genannten Mikroorganismen werden dann mit einem Vektor transformiert, der im Falle von *R. eutropha* das phaP-Gen, das für Phasin codiert und die Promotorsequenz enthält. Dieses Gen wird von einem induzierbaren Promotor, vorzugsweise einem bad-Promotor kontrolliert, der durch Arabinose induziert wird.

### Klonierungsschritte

Für die Klonierung wird die für phaP codierende DNA-Sequenz aus *R. eutropha* verwendet, die in der "GenBank" Datenbank mit der Nummer AF079155 geführt wird (Hanley, S.Z et al., FEBS Letters 1999, Vol.447, S 99-105). Diese wird in die NcoI/BamHI Schnittstelle des Vektors pBBad22K (Sukchawalit, R. et al., FEMS Microbiol Lett. 1999, Vol.181(2), S.217.223, s.a. Abb. 7) eingefügt, der einen induzierbaren Promotor (P_{BAD}) enthält. Für die Klonierung werden die entsprechenden Restriktionsschnittstellen NcoI und BamHI durch PCR-Mutagenese mittels der Primer 5'-aaaggcc**ccatgg**tcctcaccccggaaca-3' (SEQ ID Nr.1, NcoI-Schnittstelle fettgedruckt) und 5'-aaaggcc**ggatcc**tcagggcactaccttcatcg-3' (SEQ ID Nr 2, BamHI-Schnittstelle fettgedruckt) in die für Phasin codierende Sequenz eingefügt. Das dadurch enstehende DNA-Fragment der SEQ ID Nr.3 wird mit den Restriktionsenzymen NcoI und BamHI hydrolysiert und in den ebenso hydrolysierten Vektor pBBad22K (Abb 7) ligiert. Das nun die Nukleotidsequenz für das Protein Phasin enthaltende Plasmid wird im Folgenden als pBBad-P bezeichnet (Abb 8). Dieses Plasmid wird mittels der für den entsprechenden Organismus im Stand der Technik beschriebenen Transformationstechniken in den Mikroorganismus übertragen. Der Induktor des bad-Promotors des pBBad-P ist Arabinose, der es ermöglicht, die Expression dieses Gens in den verwendeten Mikroorganismen quantitativ zu kontrollieren. Über diese Steuerungsmöglichkeit wird die Expression des Phasin Gens kontrolliert und über die Menge des vorhandenen Phasins in der Zelle letztendlich die Größe der Polymerpartikel.

### Beispiel 1.2: Erzeugung von Mikroorganismen für die Herstellung von Polymerpartikeln, die ursprünglich nicht zur Bildung von Polymerpartikeln befähigt waren

Bei Mikroorganismen, die ursprünglich nicht in der Lage waren Polymerpartikel dieses Typs zu erzeugen, enthält ein zusätzlicher oder der gleiche Vektor weitere Gene, die an der Bildung der Polymerpartikel beteiligt sind. Die Anzahl und Art der erforderlichen Gene, die für die Herstellung der Polymerpartikel in einem mikrobiellen Organismus eingebracht werden müssen, bestimmen sich nach der Grundausstattung des verwendeten Organismus. Im einfachsten Fall, z.B. bei *E. coli,* ist mindestens eine Thiolase, eine Reduktase und eine Polymer-Synthase notwendig, um Polymerpartikel auf dem in Abbildung 2 dargestellten Weg herzustellen. Werden Organismen verwendet, die spezifische Mutationen aufweisen, wie zum Beispiel der *E. coli* Stamm aus Beispiel 4, reichen auch weniger als die oben genannten Gene aus, um die Herstellung der Polymerpartikel zu ermöglichen. Das gleiche gilt, wenn dem Organismus bereits bestimmte Vorstufensubstrate aus dem Polymersynthesestoffwechsel zur Verfügung gestellt werden.

Falls ein weiteres Plasmid verwendet wird um die für die Bildung der Polymerpartikel notwendigen Gene in die Zelle einzubringen, eignet sich besonders das Plasmid pBHR68 (Abb. 9), welches das 5.2 kb SmaI/EcoRI Fragment aus der chromosomalen DNA aus *R. eutropha* enthält. Dieses Plasmid enthält das Biosyntheseoperon zur Herstellung der biologisch abbaubaren Polymerpartikel aus *R eutropha* (Spiekermann, P. et al., Arch. Microbiol. 1999, Vol.171, S.73-80)

### Beispiel 1.3: Kontrolle der Partikel größe über den pBBad-P Vektor

Die so modifizierten Mikroorganismen werden bei 30°C in Luria Broth-Medium inkubiert Die Induktion des Promotors mit Arabinose erfolgt in der spätlogarithmischen Wachstumsphase. 24 h nach der Inkubation wird die Partikelgröße in den Zellen bestimmt. Dazu werden die Zellen durch Zentrifugation vom Nährmedium abgetrennt und aufgeschlossen. Danach erfolgt die mikroskopische Bestimmung der Polymerpartikelgröße mit Hilfe der Tunnelelektronenmikroskopie in Kombination mit einer analytischen Gelfiltrationschromatographie. Zur Bestimmung der Polymerpartikelanzahl werden die intakten Zellen mit einem Lichtmikroskop untersucht und die Anzahl der Polymerpartikel/Zelle ausgezählt.

Wie die Ergebnisse zeigen, ist es durch die Expressionskontrolle möglich, sowohl die Durchschnittsgröße der gebildeten Polymerpartikel (siehe Abb. 3) als auch ihre durchschnittliche Anzahl (siehe Abb. 4) in den einzelnen Zellen zu steuern. Durch kontrollierte Erhöhung der Kopienzahl von Phasin wird eine Abnahme des durchschnittlichen Polymerpartikeldurchmessers bei gleichzeitiger Zunahme der durchschnittlichen Anzahl der Polymerpartikel erreicht. So kann die Ausbeute der Polymerpartikel mit dem gewünschten Durchmesser deutlich erhöht werden. Dies ermöglicht eine schnellere und kostengünstigere Herstellung der Polymerpartikel

### Beispiel 1.3: Kontrolle der Partikelgröße über die Verfügbarkeit der Substrate

Ein weiterer Mechanismus zur Steuerung der Größe der Polymerpartikel ist die Regulation der Verfügbarkeit der Substrate oder der Polymer-Synthase, die für die Synthese der Polymerpartikel benötigt werden. Die Verfügbarkeit der Polymer-Synthase kann mittels Antisense-Technologie, durch genetische Regulation oder durch die Verfügbarkeit der Substrate, die für die Bildung der Polymer-Synthase notwendig sind, im Nährmedium gesteuert werden.

Als Beispiel für eine Regulation der Partikelgröße durch die Verfügbarkeit eines Substrates im Nährmedium dient ein Experiment, bei dem die Konzentration der vorhandenen Kohlenstoffquelle verringert wird, um so den Durchmesser der gebildeten Polymerpartikel zu steuern. Dazu wird ein *E. coli* Stamm, der das bereits zuvor genannte Plasmid pBHR68 (Abb 9) mit dem Biosyntheseoperon zur Herstellung der biologisch abbaubaren Polymerpartikel aus *R. eutropha* enthält (Abb. 8), in M9-Medium mit 1 5 (w/v) Glucose bei 30°C herangezogen. Zu Beginn der stationären Wachstumsphase wird durch Zugabe von M9-Medium ohne Glucose die Konzentration der Kohlenstoffquelle auf 1/50 seines ursprünglichen Wertes verringert und die Mikroorganismen werden bei ansonsten gleichbleibenden Wachstumsbedingungen für weitere 20 h inkubiert. Nach Beendigung des Versuches enthalten die Mikroorganismen Polymerpaitikel mit einem Durchmesser von durchschnittlich 130 nm.

### Beispiel 2: Beeinflussung der Zusammensetzung der in vivo hergestellten Polymerpartikel

Um die Zusammensetzung des biologisch abbaubaren Polymeipaitikels zu verändern, können neben dem für phaP-codierendem Gen weitere Gene, die für Enzyme codieren, die Substrate für die Synthase bereitstellen, insbesondere Thiolasen oder weitere Polymer-Synthasen, in die Zelle eingebracht werden. Dadurch ist die Zelle in der Lage, bei der Bildung der Polymerpartikel eine Reihe von unterschiedlichen Monomeren in die wachsende Polymerkette einzubauen und so Polymerpartikel herzustellen, dessen Kern aus unterschiedlich zusammengesetzten Polymeren besteht.

Als Beispiele seien hier verschiedene Polymer-Synthasen aufgezeigt, die aufgrund ihrer Substratspezifität sowohl im *in vivo* als auch im *in vitro* Verfahren unterschiedlich 3-Hydioxyfettsäuren (C4-C16) in die wachsende Polymerkette einbauen. Dabei können zum Beispiel die Polymer-Synthase aus *R. eutropha,* die biologisch abbaubare Polymerketten aus C4 Fettsäuren herstellt (C4), Polymer-Synthasen aus *Aeromonas punctata* (C4 und C6), *Thiocapsa pfennigii* (C4 und C8) und *P. aeruginosa* (C6 bis C14) verwendet werden. Auch das gleichzeitige Einbringen mehrerer Polymer-Synthasen in die Zelle, erlaubt es Polymerpartikel mit unterschiedlichsten Eigenschaften herzustellen. In einem weiterführenden Ansatz können durch Zufallsmutagenese (*in vitro* evolution) auch neue Polymer-Synthasen mit veränderter Substratspezifität gewonnen werden.

Die Zusammensetzung der sich bildenden Polymerpartikel lässt sich auch noch auf anderem Wege beeinflussen. Die Bereitstellung von unterschiedlichen Kohlenstoffquellen, Vorstufen verschiedener Kohlenstoffquellen und Intermediaten des Stoffwechselweges, die zur Bildung der Polymerpartikel führt, haben ebenfalls Einfluss auf die Art des sich bildenden Polymers. Des Weiteren können die an der Bildung der Polymerpartikel beteiligten Stoffwechselwege durch Inhibitoren, Verwendung von *Knock-out* Mutanten der betreffenden Stoffwechselwege und der Expression von Enzymen, welche im Stoffwechselweg zur Bildung anderer Zwischen- oder Endprodukte führen, gesteuert werden.

Für die Beeinflussung des Fettsäurestoffwechsels werden die folgenden Enzyme eingesetzt, welche die Eigenschaft haben, die Zwischenprodukte des Fettsäunestoffwechsels zu modifizieren und so unterschiedliche Produkte, z B. Fettsäuren mit unterschiedlichen Seitenketten, für die Bildung der Polymerpartikel zur Verfügung zu stellen: (R)-spezifische Enoyl-CoA Hydratasen, Transacylasen und Ketoacyl-CoA/ACP Reduktasen. Diese Enzyme haben eine unterschiedliche Spezifität im Bezug auf die Kettenlänge des für die Polymer-Synthase beteitgestellten Substrates (R)-Hydroxyacyl-CoA Dadurch entstehen Bausteine im Polymer mit verschiedenen Seitenkettenlängen und somit Polymere unterschiedlicher Zusammensetzungen.

Die Beeinflussung des Stoffwechselweges kann aber nicht nur über das Einbringen von zusätzlichen Enzymen in die Zelle erfolgen sondern auch über den Zusatz von Inhibitoren des Stoffwechselweges zum Medium Beispiele für solche Inhibitoren sind die Acrylsäure und Triclosan (Synonyme: TCC oder 5-Chlor-2-(2,4-dichlorphenoxy)-phenol), um nur einige zu nennen.

### Beispiel 3: In vivo Herstellung von biologisch abbaubaren Polymerpartikeln aus (R)-3-Hydroxybuttersäure mit hoch kristallinem Kern

Für dieses Experiment wurde ein *E. coli* Stamm verwendet, der das bereits im Beispiel 1 genannte Plasmid pBBad-P und das Plasmid pBHR68 enthält. Die Kultivierung erfolgte unter den in Beispiel 1 genannten Bedingungen mit Glucose als Kohlenstoffquelle. Die gebildeten Polymerpartikel bestehen aus (R)-3-Hydroxybuttersäure und haben einen Durchmesser von 50 bis 500 nm, je nachdem wie viel Induktor (Arabinose) dem Medium zugegeben wird. Die Durchschnittliche Größe der so gebildeten Polymerpartikel schwankt dabei nur um etwa 20 bis 50 nm. Als Verdeutlichung der Kontrolleigenschaften, die durch das über das pBBad-P Plasmid eingeführte Phasin erzielt werden, wird das Experiment mit dem oben genannten *E*. *coli* Stamm ohne das pBBad-P Plasmid wiederholt, Hierbei werden Polymeipaitikel mit einem Durchmesser von 150 - 250 nm erhalten, Die Steuerung der Partikelgröße über das Phasin ermöglicht im Vergleich zum Kontrollexperiment die Herstellung viel größerer aber vor allem auch viel kleinerer Polymerpartikel.

Eine weitere Fraktionierung der Polymerpartikel nach ihrer Größe kann anschließend durch im Stand der Technik bekannte Verfahren, wie z.B, der Ausschlußchromatographie, der Dichtegradientenzentrifugation, oder der Ultrafiltration in 5 mM Phosphatpuffer (pH 7 5) erfolgen.

### Beispiel 4: In vivo Herstellung von biologisch abbaubaren Polymerpartikeln aus (R)-3-Hydroxyfettsäuren mit niedrig kristallinem Kern

In diesem Experiment werden biologisch abbaubare elastomere Polymerpartikel in *E. coli* hergestellt, deren Kerne aus (R)-3-Hydroxyfettsäuren bestehen. Die Polymerketten sind im Durchschnitt aus 6 bis 14 Kohlenstoffatomen aufgebaut Dies wird durch die regulierte Expression des phaF-Gens aus *P. oleovorans* in *E coli* erreicht, dass dem für Phasin codierendem Gen phaP aus *R eutropha* sehr ähnlich ist und dem Gen für die Polymer-Synthase aus *P aeruginosa* (phaC). In diesem Beispiel wird das Expressionsprodukt des Gens phaF zur Kontrolle der Partikelgröße eingesetzt. Die Polymerpartikel können in *E coli*-Zellen mit verändertem Fettsäuremetabolismus alleine durch die Polymer-Synthase (phaC) der Pseudomonaden hergestellt werden (Langenbach, S, et al., FEMS Microbiol. Lett 1997, Vol 150, S.303-309). Der Fettsäuremetabolismus ist so verändert, dass bei Verwendung von Fettsäuren als Kohlenstoffquelle CoA-aktivierte Intermediate (z,B Enoyl-CoA) der Fettsäure-ß-Oxidation akkumuliert werden, die wiederum als Vorstufen der Polymersynthese dienen. Dafür werden fadB-Mutanten von *E. coli* verwendet (Langenbach, S. et al,, FEMS Microbiol., Lett. 1997, Vol 150, S 303-309) oder es werden. Inhibitoren eingesetzt, welche den Fettsäuremetabolismus entsprechend inhibieren (z. B. Acrylsäure; Qi, Q. et al , FEMS MicrobioL Lett. 1998, Vol 167, S 89-94). Die ansonsten benötigte Ketoacyl-Reduktase und die Thiolase sind bei Verwendung der Mutanten oder inhibierten Milaoorganismen nicht mehr erforderlich. *E. coli* eigene Enoyl-CoA-Hydiatasen katalysieren dann die Bildung von R-3-Hydroxyacyl-CoA aus Enoyl-CoA, Dies wird dann von der Polymer-Synthase in Poly(R)-3-Hydroxyfettsäure umgewandelt, welches den Polymerkern des sich bildenden Polymerpartikels bildet.

### Klonierung

Das phaF-Gen von *P. oleovorans,* dass bereits von Prieto et al. (prieto, M.A. et al., J. Bacteriol 1999, Vol 181(3), S 858-868) beschrieben worden ist und in der "GenBank" Datenbank mit der Nummer AJ010393 geführt wird, wird über den Vektor pBBad-F (Abb. 11) in *E. coli* transformiert, Dieser Vektor wird ausgehend vom Vektor pBBad22K erhalten (Abb 7) Die einzelnen Klomerungsschritte entsprechen dabei den im Beispiel 1 beschriebenen. Das phaF-Gen wird in die NcoI/BamHI Schnittstelle des Vektors pBBad22K kloniert. Die Primer, die zur PCR-Mutagenese des oben genannten phaF-Gens verwendet werden, haben die folgenden Sequenzen: 5'-aaaggg**ccatgg**ctggcaagaagaattccgagaa-3' (SEQ ID Nr 4, Ncol-Schnittstelle fettgedruckt) und 5' -aaaggg**ggatcc**tcagatcagggtaccggtgcctgtctg-3' (SEQ ID Nr. 5, BamHI-Schnittstelle fettgedruckt). Das dadurch entstehende DNA-Fragment der SEQ ID Nr. 6 wird dann in das bereits beschriebene Plasmid pBBad22K kloniert, Das nun die Sequenz für phaF enthaltende Plasmid wird im folgenden als pBBad-F bezeichnet (Abb 11). Zusätzlich zu diesem Plasmid wird auch das die Nukleotidsequenz für die Polymer-Synthase enthaltende Plasmid pBHR71 (Abb. 10; Langenbach, S et al., FEMS Microbiol Lett. 1997, Vol.150. S 303-309) in *E. coli* transformiert.

Als Kohlenstoffquelle für die anschließende Expression, wird die Fettsäure Decansäure verwendet Die Expression des Phasin phaF-Gens wird ebenfalls über den Induktor Arabinose kontrolliert, Der Versuch wird dabei, wie bereits in Beispiel 1 beschrieben, durchgeführt. Die so gebildeten Polymerpartikel haben je nach Menge des zuvor eingesetzten Induktors einen Durchmesser von 100-500 nm

### Beispiel 4: Kontrolle der Größe von in vitro hergestellten biologisch abbaubaren Polymerpartikel

Die Kontrolle der Größe der gebildeten biologisch abbaubaren Polymerpartikel wird auch bei der *in vitro* Herstellung durch die Verfügbarkeit der Polymer-Synthase, von Phasin oder Phasin-ähnlicher Proteine und die Verfügbarkeit der Substrate und Stoffwechselintermediate bestimmt.

Für den *in vitro* Ansatz zur Herstellung der biologisch abbaubaren Polymerpartikel, müssen zuerst die notwendigen Enzyme und Substrate zur Verfügung gestellt werden. Für diesen Versuch wird die rekombinante Polymer-Synthase aus *R*. *eutropha* oder *P. aeruginosa* verwendet (Gemgross, T U und Martin, D P., Proc. Natl. Acad, Sci. USA 1995, VoL92, S.6279-6283; Qi, Q et al,, Appl. Microbiol. Biotechnol 2000, Val. 54, S 37-43), die mittels Affinitätschromatographie (mit His-Tag-Fusion oder Ni²⁺-NTA-Agarose) gereinigt werden Analog dazu werden für die Kontrolle der Paitikelgröße jeweils die Polymerpartikelgröße-bestimmenden Proteine aus *R. eutropha* bzw. *P. aeruginosa* aufgereinigt, Zun Expression dieses Proteins in *R. eutropha* wird der Vektor verwendet, der bereits im Beispiel 1 zur Expression des Phasin-Gens verwendet worden ist Der Reaktionsansatz zur *in vitro* Herstellung der biologisch abbaubaren Polymerpartikel enthält neben der Polymer-Synthase und dem Polymerpartikelgröße-bestimmenden Protein Phasin weiterhin; R-3-Hydroxybutyryl-CoA oder R-3-Hydroxydecanoyl-CoA als Substrat für die Synthese der Polymerpartikel, 50 mM Phosphat-Puffer (pH 7.5), 1 mM MgCl₂ und zur Stabilisierung 5% Glycerol (v/v). Aufgrund der Verwendung von R-3-Hydroxybutyryl-CoA oder R-3-Hydroxydecanoyl-CoA als Vorstufenprodukte für die Polymer-. Synthase ist der Einsatz der Thiolase und der Reduktase nicht erforderlich (s.a. Abb. 2)

Die Ergebnisse der Abb 5 stellen den Einfluss des Mengenverhältnisses von Phasin zur Polymer-Synthase auf den Polymerpartikeldurchmesser dar, während Abb. 6 den Einfluss des Verhältnisses von Substrat zur Polymer-Synthase auf den Polymerpartikeldurchmesser darstellt. Diese Ergebnisse zeigen auch, dass sich die Regulation der Polymeipartikelgröße über die Menge der vorhandenen Polymei-Synthase regulieren lässt. Somit kann über das erfindungsgemäße Verfahren eine effektive Regulation der Partikelgröße erzielt werden.

### Beispiel 5: Beeinflussung der Zusammensetzung der in vitro gebildeten Polymerpartikel

### Beispiel 5.1: Beeinflussung der Polymerzusammensetzung

Um die Zusammensetzung des biologisch abbaubaren Polymerpartikels zu verändern, können dem Reaktionsansatz verschiedene Substrate zugegeben werden und/oder jeweils verschiedene Polymer-Synthasen mit unterschiedlichem Substratspektrum verwendet werden (s.a Beispiel 2). Dadurch werden bei der Bildung der Polymerpartikel eine Reihe von unterschiedlichen Monomeren in die wachsende Polymerkette eingebaut und es werden Polymerpartikel mit unterschiedlicher Polymerzusammensetzung hergestellt. Durch die Verwendung der Polymer-Synthase aus *P aeruginosa* werden R-3 Hydroxyfettsäure-Bausteine mit 6-14 C-Atomen in die wachsende Polymerkette eingebaut. Wird z.B, nur ein Substrat angeboten, so werden bei Verwendung der Polymer-Synthase aus *P. aeruginosa* homopolymere Polymerpartikel gewonnen,

Weitere Beispiele, wie die Zusammensetzung der Polymerpartikel verändert werden kann, finden sich in Beispiel 2.

### Beispiel 5.2: Beeinflussung der Membranzusammensetzung der Polymerpartikel

Für eine spätere Fusion mit der Membran einer Zelle, in der z B ein Wirkstoff eingebracht werden soll, bedarf es der Möglichkeit die Zusammensetzung des Phospholipidschicht auf der Oberfläche der biologisch abbaubaren Polymerpartikel zu kontrollieren. Diese Regulation erfolgt bei der *in vitro* Herstellung (Beispiel 4) der biologisch abbaubaren Polymerpartikel bereits bei der Bereitstellung der für die Polymerpartikelbildung geeigneten Lösung Durch Zugabe eines Gemisches verschiedener amphipiler Moleküle, kann die Membran den entsprechenden Erfordernissen angepasst werden

Zum *in vitro* Reaktionsansatz weiden Phospholipide hinzugegeben. Eine definierte Anzahl von Molekülen der Polymer-Synthase sind nun an der Entstehung der Polymerpartikel beteiligt Die erfindungsgemäßen Polymerpartikel werden mit zunehmender Polymerisation größer und die Polymere Synthasen an der Oberfläche, können die Oberfläche der Polymerpartikel nicht mehr vollständig abschirmen Folglich weiden hydrophobe Bereiche (Polymer) exponiert, an die sich amphiphile Moleküle spontan anlagern.

Bei den *in vivo* hergestellten Partikeln muss zunächst die bereits vorhandene Phospholipidschicht entfernt werden., Dafür wird eine Aceton-Extraktion durchgeführt oder es werden Phospholipasen oder nichtdenaturierenden Detergentien verwendet, um die Phospholipidschicht zu zerstören. Danach wird, wie bereits oben beschrieben, eine Mischung der entsprechenden amphiphilen Moleküle zu den nun nahezu Lipid-freien Polymerpartikeln gegeben. Bevorzugt werden dabei negativ geladene Phospholipide oder Phosphatidylcholin verwendet. In einem Ausführungsbeispiel für die gezielte Steuerung der Membranzusammensetzung weiden die Polymeipaitikel in 1% Octylglycosid-haltigen PBS (pH 7.5) suspendiert und unter Rühren gegen einen Überschuss Phosphatidylcholin dialysiert. Die so erzeugte Partikeloberfläche eignet sich besonders gut für die Fusion mit Hirnkapillar-Endathelzellen (BCEC).

### Beispiel 5.3: Einbau verschiedener Substanzen in die wachsenden Polymerpartikel

Mit Hilfe des lipophilen Fluoreszenzfarbstoffes Nilrot (Sigma, St Louis, Mo , USA) bzw. Rhodamin 123 wurde bereits die Aufnahme von Substanzen in den Kern des biologisch abbaubaren Polymerpartikels qualitativ untersucht (Spickeimann, P. et al, Arch. Microbiol 1999, Vol.171, S.73-80). Wenn bei der *in vivo* oder der *in vitro* Herstellung, wie oben beschrieben, der Fluoreszenzfarbstoff Nilrot ins Medium oder dem Reaktionsansatz gegeben wird, kann eine Färbung der hergestellten Polymerpartikel beobachtet werden, d.h, die Färbung der Polymerpartikel tritt bereits während ihrer Synthese und sogar vor der Isolation aus der Zelle ein,

Durch Zugabe pharmazeutisch aktiver Wirkstoffe können diese in den Polymerkern des Partikels eingebaut werden. Zum Beispiel wird das unpolare anti-Tumormittel Paclitaxel bei einem *in vitro*

Experiment zur Herstellung der Polymerpartikel zum Reaktionsansatz zugegeben Das wasserunlösliche Paclitaxel löst und konzentriert sich im hydrophoben Polymer-Kern der Polymerpartikel. Paclitaxel wird der Lösung für die Bildung der Polymerpartikel dabei lediglich zugesetzt, woraufhin sich der Wirkstoff in den Partikeln konzentriert Dies wird überprüft, indem nach der Bildung der Polymerpartikel diese aus dem Reaktionsansatz entfernt werden und die Lösung mittels HPLC auf die Anwesenheit von Paclitaxel untersucht wird Die Abnahme der Paclitaxel-Konzentration in der Lösung zeigt dabei, dass es in die Polymerpartikel eingebaut worden ist. Mit dem biologischen Abbau der Polymerpartikel im Organismus erfolgt später die Freisetzung des Wirkstoffs, Als Kontrollversuch dient ein Reaktionsansatz ohne Polymerpartikel, wobei hier die Paclitaxel-Konzentration in der für die Polymerpartikelbildung geeigneten Lösung nicht abgenommen hat

### Beispiel 6: Funktionalisierung der Partikeloberfläche

Biologisch aktive Substanzen können an Proteine gebunden werden, die sich bereits auf der Oberfläche der Polymerpartikel befinden Dafür kommen alle in Abbildung 1 genannten Proteine in Betracht. Dadurch ergeben sich eine Vielzahl von *"cross-linking"*-Strategien, die eine kovalente Verknüpfung der biologisch aktiven Substanz über Proteine, die an die Partikeloberfläche gebunden sind, ermöglichen.

### Beispiel 6.1 Gezielte Modifikation eines Oberflächenproteins zur Anbindung eines pharmazeutischen Wirkstoffes

Ein Beispiel für die Funktionalisierung der Polymerpartikel ist die Anbindung von Hydrazongebundenem Doxorubicin (Hydrazon-gebundenes Doxorubicin wurde beschrieben von King, H D. et al., Biconjugate Chem 1999, Vol.10, S 279,288) an die Polymer-Synthase PhaCl aus *P*. *aeruginosa,* die auf der Oberfläche der Polymerpartikel gebunden ist und die zwei N-terminal eingefügte Cystein-Reste enthält Diese Cystein-Reste bilden die Bindedomäne der Polymer-Synthase, über die biologisch aktive Substanzen gebunden werden können.

### Klonierung

Die Cystein-Reste codierenden Tripletts weiden mittels PCR-Mutagenese in das für PhaCl codierende Gen kloniert, dass dann in die XbaI und BamHI Schnittstelle des in Abbildung 10 dargestellten Plasmids pBHR71 kloniert wird. Bei dieser PCR-Mutagenese werden auch die Schnittstellen für XbaI und BamHI in das Gen eingefügt Die Primer, die zur PCR-Mutagenese des für PhaCl codierende Gens verwendet werden, haben die folgenden Sequenzen: Primer für den N-Ierminus 5'-gggc**tctaga**aataaggagatatacatatgtgttgtaagaacaataacgagctt-3' (SEQ ID Nr.7, XbaI-Schnittstelle fettgedruckt, Cys-Tripletts unterstrichen) und Primer für den C-Terminus 5'-,aaacgc**ggatcc**ttttcatcgtteatgca-3' (SEQ ID Nr.8, BaniHI-Scbnittstelle fettgedruckt), Das dadurch entstehende DNA-Fragment der SEQ ID Nr. 9 wird dann mit XbaI und BamHI hydrolysiert und in das analog hydrolysierte Plasmids pBER71 ligiert Das daraus resultierende Plasmid, genannt pBHR71-Cys (Abb 12), wird mittels bekannter Techniken in *E. coli* transformiert, wo es dann die Bildung von Polymerpartikeln ermöglicht, die auf ihrer Oberfläche eine Polymer-Synthase tragen, die zwei Cystein-Reste für die Anbindung von diversen Substanzen, insbesondere pharmazeutischen Substanzen trägt,

### Beispiel 6.1.1 Anbindung von Doxombicin (Syn.: Hydroxyl daunorubicin)

Über diese Oberflächen-exponierten Cystein-Reste kann nun ein Hydrazon-vermitteltes *"cross-linking"* erfolgen. Dazu wird wie folgt vorgegangen: 100 mg des isolierten Polymerpartikels (Gesamtvolumen 1 ml) auf dem die Polymel-Synthase gebunden ist, werden mit Helium-begastem PBS (pH 7.5) und 5 mM Dithiothreitol (DTT) für 3 h bei 37°C inkubiert. Durch diese Behandlung weiden die Disulfidbrücken in der Polymer-Synthase reduziert Danach werden durch eine 30 min Zentrifugation bei 4°C und 40,000 x g die niedermolekularen Verbindungen entfernt. Dann werden die reduzierten Polymerpartikel mit 1 ml PBS-Puffer (pH 7.5), der 10 µmol des Hydrazon-gebundenen Doxorubicin enthält, suspendiert und für 30 min bei 4°C inkubiert. Danach findet eine erneute Zentrifugation unter den oben genannten Bedingungen statt, um die behandelten Polymerpartikel zu waschen. Durch eine anschließende HPLC wird ungebundenens Doxorubicin nachgewiesen und durch die verringerte Konzentration somit die erfolgreiche Anbindung an die Polymer-Synthase überprüft.

### Beispiel 6.2 Anbindung von biologisch aktiven Substanzen, insbesondere pharmazeutischen Wirkstoffen an die Bindedomäne der Polymerpartikel

Wirkstoffe können ebenso an die Polymerpartikel gebunden werden, indem sie über die Bindedomäne an die auf der Oberfläche der Polymerpartikel gebundenen Proteine gebunden werden. Dazu muss erst einmal eine Bindedomäne geschaffen werden. Die Bindung von biologisch aktiven Substanzen wird durch genetische Modifikation der auf der Oberfläche gebundenen Proteine der Polymerpartikel (wie z.B. Polymer-Depolymerase, Phasin oder Phasin-ähnliche Proteine, Polymer-Synthase, Polymer-Regulator) erreicht, so dass diese Proteine eine nach außen gerichtete Bindedomäne ausbilden, über die ein Kopplungsreagenz oder eine biologisch aktive Substanz gebunden werden kann. Bei der Fusion der oben genannten Oberflächenproteine der Polymerpartikel mit einem Protein, das die Anbindung eines Kopplungsreagenzes oder einer biologisch aktiven Substanz direkt ermöglicht, ist bei diesem Verfahren darauf zu achten, dass nach der Fusion mit dem Oberflächenprotein des Polymerpartikels die Funktionalität sowohl des Oberflächenproteins als auch des fusionierten Proteins voll erhalten bleiben muss.

In einem Ausfüungsbeispiel wird ein Polymerpartikel mit zwei FLAG-Epitope direkt an den N-Terminus der Polymer-Synthase PhaC1 aus *P. aeruginosa* fusioniert. Die FLAG-Bpitope ermöglichen die Bindung von kommerziellen anti-FLAG mAbs (monoklonale Antikörper) (Anti-FLAG M2, Sigma-Aldrich) und anschließend von Enzymmarkern, welche die erfolgreiche Durchführung des Verfahrens belegen sollen. In diesem Beispiel werden sekundären Antikörper-alkalische Phosphatase Konjugate (Anti-Mouse-Alkalische Phosphatase, Sigma-Aldrich) als Enzymmarker verwendet. Die Aktivität der alkalischen Phosphatase an der Oberfläche der Polymerpartikel wird anschließend photometrisch bestimmt.

### Beispiel 6.2.1 Herstellung eines Polymerpartikels mit einem FLAG-PhaC1=Fusionsprptein

Für die Herstellung des FLAG-Polymer-Synthase-Fusionsproteins werden die folgenden Oligonukleotide verwendet: 5'-tatg**actagt**gattataaagatgatgatgataaaca-3' und 5'tatgtttatcatcatcatctttataatc**actagt**ca-3' (SEQ ID Nr. 10 bzw SEQ ID Nr. 11, SpeI-Schnittstelle fettgedruckt, FLAG-Epitop unterstrichen). Um durch Hybridisierung doppelsträngige DNA zu erhalten, werden beide Oligonukleotide in äquimolarer Menge (je 10 µM) miteinander vermischt und für 30 min bei Raumtemperatur (RT) inkubiert. Die dadurch gebildete doppelsträngige DNA codiert für das FLAG-Epitop (DYKDDDDK) und besitzt überhängende Enden (TA), die mit den überhängenden Enden der NdeI Schnittstelle komplementär sind. Dieses DNA-Fragment wird mit dem Restriktionsenzym NdeI hydrolysiert und in den analog dazu ebenfalls hydrolysierten Vektor pBHR71 (Abb 10) kloniert. Das so entstehende Plasmid pBHR71-FLAG (Abb. 13) enthält das Gen mit der SEQ ID Nr. 12 und vermittelt die Expression einer Polymer-Synthase mit N-terminaler FLAG-Fusion (dieser Teil des Proteins bildet nun die Bindedomäne). Über diese Bindedomäne können nun biologisch aktive Substanzen und/oder Kopplungsreagenzien gebunden werden. Die bei der Klonierung ebenfalls eingefügte singuläre SpeI-Schnittstelle steht zusätzlich für die Insertion beliebiger weiterer DNA-Fragmente, die für Funktionsproteine codieren zur Verfügung.

Als Beispiel für die Funktionalität des zuvor geschilderten Konstruktes wird wie folgt vorgegangen Die Polymerpartikel werden nach Transformation des pBHR71-FLAG Plasmids in *E. coli* Stämme, die bereits das Plasmid pBBad·F enthalten und einen veränderten Fettsäuremetabolismus aufweisen (s Beispiel 3) exprimiert. Durch Aufschluss der Zellen werden die Polymerpartikel aus den Zellen isoliert und dreimal mit PBS-Puffer (pH 7.5) gewaschen. Dann werden die Polymerpartikel für 30 min bei RT mit monoklonalen anti-FLAG-Antikölpern inkubiert, die an die FLAG-Epitope binden. Danach werden die Polymerpartikel erneut, wie bereits oben beschrieben, gewaschen und anschließend mit sekundärem alkalische Phosphatase Konjugat für 30 min bei RT in PBS-Puffer inkubiert Nach der 30 min Inkubation werden diese Partikeln in 0.1 M Tris-HCI (pH 8 5) gewaschen und dann werden der Paitikelsuspension als Substrat für die alkalische Phosphatase 2 mg/ml p-Nitrophenylphosphat zugesetzt Die Aktivität der alkalischen Phosphatase wird spektrometrisch bei 410 nm gemessen. Als Negativ-Kontrolle dienen Polymerpartikel, die eine Polymer-Synthase ohne Bindedomäne, d.h. ohne FLAG-Epitop enthalten. Aufgrund der fehlenden Umsetzung des zugegebenen p-Nitrophenylphosphates in der Kontrolle ergeben die spektrometrischen Messungen bei 410 nm ein negatives Ergebnis Die Tatsache, dass die Polymerpartikel sich überhaupt gebildet haben, ist ein Beleg dafür, dass der Einbau des FLAG-Epitopes keine Auswirkung auf die Funktionalität der Polymer-Synthase gehabt hat.

Dieses Verfahren kann auch mit einem der anderen bereits genannten Oberflächenproteine der Polymerpartikel durchgeführt werden Werden mehrere Oberflächenproteine gleichzeitig modifiziert, können eine Vielzahl unterschiedlichster Substanzen an die Polymerpartikel gebunden weiden und somit eine Multi·Funktionalität ermöglichen, die sie vielfältig einsetzbar macht

Während im vorigen Beispiel eine biologisch aktive Substanz nachträglich an das bereits exprimierte und gebildete Oberflächenprotein des Polymerpartikels gebunden worden ist, besteht natürlich auch die Möglichkeit, das Protein direkt mit dem Qberflächenprotein zu fusionieren und dann zu exprimieren. Dazu werden die codierenden Sequenzen der Proteine (z.B. Enzyme) mit dem C-terminalen Ende des phaC1 Gens des pBHR71-FLAG Plasmids fusioniert.

Durch PCR werden DNA-Fragmente gewonnen, die jeweils für das einzufügende Protein und für den C-tetminalen Abschnitt der Polymer-Synthase codieren, um eine Fusion mit dem phaC1-Gen zu ermöglichen. Beide Fragmente weiden über eine mit überhängenden Primern eingefügte Restriktionsschnittstelle miteinander ligiert. Am 5'-Ende dieses Hybrid-Genes wird im Abstand von 7 nt zum Startcodon eine ribosomale Bindestelle (GAGGAG) und eine Restriktionsschnittstelle mittels überhängender Primer eingefügt. Wenn der verwendet Vektor, wie z.B. der hier verwendete Vektor pBHR71 -FLAG, bereits eine ribosomale Bindestelle besitzt, ist das zusätzliche Einfügen einer ribosomalen Bindestelle nicht mehr erforderlich. Zusammen mit einer eingefügten Restrktionsschnittstelle am 3'-Ende dieses Hybrid-Genes ist nun die gezielte Klonierung in den Expressionsvektor pBHR71-FLAG möglich. Die Restriktionsschnittstellen am 5' und 3' Ende des Hybrid-Genes müssen so gewählt werden, dass diese innerhalb des Hybrid-Genes nicht ein zweites mal vorkommen, damit eine Klonierung in einen Expressionvektor colinear zum vorliegenden Promotor erfolgen kann. In diesem Beispiel wird das lacZ-Gen aus *E. coli* mittels PCR mit Primern amplifiziert, die eine SpeI-Schnittstelle enthalten: 5'-gg**actagt**atgaecatgattaeggattcactggc-3' (SEQ ID Nr.13, SpeI-Schnittstelle fettgedruckt) und 5'-cc**actagt**tttttgacaccagaccaactggtaatggtagcg-3' (SEQ ID Nr.14, SpeI. Schnittstelle fettgedruckt). Zusätzlich wird das Stop-Codon durch Verwendung dieser Primer aus der Sequenz des lacZ-Gens entfernt, um einen durchgehenden Leserahmen zu erhalten. Das daraus resultierende DNA-Fragment mit der SEQ ID Nr 15 wird direkt in die SpeI-Schnittstelle des pBHR71-FLAG Plasmids kloniert Das gewonnene Fusionsprotein führte zur Bildung von Polymerpartikel mit β-Galactosidase-Aktivität. Die entsprechenden Polymerpartikel werden isoliert und unter reduzierenden Bedingungen wird die β -Galactosidase-Aktivität mit dem Substrat o-Nitrophenyl-beta-D-galactopyranosid (Calbiochem) nachgewiesen.

### Beispiel 7: Stabilität der Bindung zwischen den Oberflächenproteinen und dem Polymerkern der Polymerpartikel

Im Rahmen der Erfindung getätigte Untersuchungen haben ergeben, dass die Polymer-Synthase weder durch Behandlung mit denaturierenden Reagenzien, wie z.B. Sodiumdodecylsulfat (SDS), Harnstoff, Guanidiumhydrochlorid oder Dithiothreitol, noch durch Verwendung azider Bedingungen vom Kern des biologisch abbaubaren Polymer-Partikels gelöst werden kann. Dies deutet auf' eine kovalente Verknüpfung hin, die zwischen dem Polymerpartikel und der Polymerpartikelbindedomäne der Polymer-Synthase besteht.. Die hohe Stabilität der Bindung ermöglicht einen stabilen Transport von gebundenen oder in die Polymerpartikel eingebaute Substanzen an ihren Zielort. Der N-terminale Abschnitt der Obeiflächen-gebundenen Polymer-Synthase (N-Terminus bis zum Beginn der konservierten a/β-Hydrolase-Domäne) ist äußerst variabel und kann durch gentechnische Verfahren durch Funktionsproteine ersetzt werden. Dabei bleibt die Polymer-Synthase-Aktivität, und Synthese von Polymerpartikeln erhalten (Rehm, B.H.A. et al., Biochem Biophys. Acta 2002, Vol.1594, S.178-190) Folglich wird eine Funktionalisierung der Oberfläche erreicht, die eine hohe Stabilität aufweist Ein Gemisch unterschiedlicher Proteine mit unterschiedlichen Bindedomänen, an die biologisch aktive Substanzen und/oder Kopplungsreagenzien gebunden sind, werden bei Bedarf gleichzeitig appliziert werden, so dass eine Multifunktionansierung der Oberfläche der Polymerpartikel erfolgt Die Applikation dieser Proteine erfolgt *in vitro* durch Zugabe der gereinigten Proteine mit unterschiedlichen Bindedomänen zum Synthese-Ansatz bzw. *in vivo* durch Expression der Gene in dem entsprechenden Mikroorganismus, die für jeweils ein Protein mit einer Bindedomäne codieren.

### Beispiel 7.1 Weitere Möglichkeiten für eine Modifikation der Oberflächenproteinen der Polymerpartikel

Der C-terminale Abschnitt des Oberflächenproteins Phasin (PhaP) aus *R. eutropha* (Aminosämereste ab >Ala141) ist hydrophil und kann durch Funktionsproteine ersetzt werden, ohne eine Verankerung des Phasins über die Polymerpartikelbindedomäne an die Oberfläche der Polymerpartikel zu verhindern

Diese Verankerung beruht auf hydrophoben Wechselwirkungen und ist reversibel (Hanley, S.Z et al., FEBS Letters 1999, Vol.447, S.99-105) Dieser C-terminale Abschnitt der intrazellulären Polymer-Depolymerasen wird durch gentechnische Verfahren mit Funktionsproteinen fusioniert und ermöglicht somit eine Funktionalisierung der Oberfläche des Polymerpartikels über eine anschließende Anbindung von biologisch aktiven Substanzen und/oder Kopplungsreagenzien

Der C-Terminus (Aminosäurerest ab >180) der intrazellulären Polymer-Depolymerase von *R. eutropha* vermittelt die Bindung des Enzyms an den Kern der Polymerpartikel (Saegusa, H et al., J. Bacteriol. 2001, Vol.183(1), S.94-100). Dieser C-terminale Abschnitt der intrazellulären Polymer Depolymerasen wird durch gentechnische Verfahren mit Funktionsproteinen fusioniert und ermöglicht somit eine Funktionalisierung der Oberfläche des Polymerpartikels

Der N-Terminus (Ammosäurerest ab <140) der an die Oberfläche des Polymerpartikels gebundenen Expressionsprodukte der *Gene phaI* und *phaF* aus *Pseudomonas oleovorans* vermittelt die Bindung der Proteine an den Polyester-Kern der Polymerpartikel (Prieto, M.A. et al., J Bacteriol. 1999, Vol.181(3), S. 858-868). Dieser N-terminale Abschnitt der Expressionsprodukte der Gene phaF und phaI wird durch gentechnische Verfahren mit einem Funktionsprotein fusioniert und die dadurch entstehende Bindedomäne ermöglicht anschließend eine Funktionalisierung der Oberfläche des Polymerpartikels über die Anbindung von biologisch aktiven Substanzen und/oder Kopplungsreagenzien.

### Beispiel 8: Kovalente Modifikation der Oberflächenproteine der Polymerpartikel mit einem Kopplungsreagenz

Die in Abbildung 1 dargestellten Proteine auf der Oberfläche der biologisch abbaubaren Polymeipartikel können mit speziellen Markierungssubstanzen behandelt werden, die spezifisch an bestimmte Aminosäuren binden (z.B. N-Hydroxysuccinimid-Biotin an Lysin). Dies ermöglicht das Anbinden von biologisch aktiven Substanzen, wie z.B. von Biotin über die Iodacetamid vermittelte Verknüpfung an Cystein. Moleküle, wie Biotin, vermitteln dann eine weitere Verknüpfung von biologisch aktiven Substanzen and die Oberflächenproteine dieser Polymerpartikel. Dazu gehören z.B. Avidin oder Streptavidin, die selbst an Enzyme gebunden sein können und so eine schrittweise Funktionalisierung der Oberflächenproteine der biologisch abbaubaren Polymerpartikel erlauben (Rehm, B.H.A. et al., J. Bacteriol. 1994, Vol.176, S.5639-5647). Diese Funktionalisierung kann durch Anbinden von Antikörpern oder pharmazeutischen Wirkstoffen erfolgen. Es können auch Moleküle mit unterschiedlichen Oberflächenladungen angebracht werden, um dem Polymerpartikel eine bestimmte Ladung auf der Oberfläche zu verleihen, die für den Transport und die Fusion über/mit bestimmten Membranen vorteilhaft ist.

Eine Markierung von Lysinresten an den Oberflächenproteinen der Polymerpartikel mit Biotin, wird mittels N-Hydroxysuccinimid-Biotin erzielt. Bei diesem Experiment werden Polymerpartikel, welche die Polymer Synthase PhaC1 aus *P. aeruginosa* tragen, aus rekombinanten *E coli* isoliert, welches das Plasmid pBHR71 trägt. Die Polymerpartikel werden nach der Isolation dreimal in PBS (pH 8.0) gewaschen und N-Hydroxysuccinimid-Biotin (Sigma-Aldrich) wird dann der Lösung bis zu einer Endkonzentration von 5 mM hinzugegeben Die Reaktion wird durch erneutes Waschen nach 5 min Inkubation bei 4°C beendet. Der Nachweis des an die Partikeloberfläche-gebundenen Biotins wird mit Hilfe des Streptavidin-Alkalische-Phosphatase-Konjugates (Sigma-Aldrich) und o-Nitrophenylphosphat (Calbiochem) als Substrat nachgewiesen Im Kontrollansatz mit Partikeln, die nicht mit N-Hydroxysuccinimid-Biotin behandelt worden sind, zeigen diese keine alkalische Phosphatase-Aktivität.

Neben den hier aufgeführten Beispielen gibt es für die Anknüpfung von biologisch aktiven Substanzen noch viele Kopplungsreagenzien, mit deren Hilfe die Obierflächenproteine der hier hergestellten Polymerpartikel aktiviert werden können.

Die Erfindung wird nun durch die folgenden Bedingungen definiert:
1. Verfahren zur Herstellung von biologisch abbaubaren Polymerpartikeln, das umfasst;
   a) Einbringen zumindest eines induzierbaren Gens in einen Mikroorganismus, wobei das Gen für ein Protein codiert, das die Größe der Polymerpartikel kontrolliert; und
   b) Kultivieren des Miktoorganismus unter Induktion des unter a) genannten zumindest einen induzierbaren Gens in einem Kulturmedium unter Bedingungen, die zur Herstellung der biologisch abbaubaren Polymerpartikel durch den Mikroorganismus geeignet sind.
2. Verfahren nach Bedingung 1, wobei man in Stufe a) neben dem zumindest einem induzierbaren Gen, das für ein Protein codiert, das die Größe der Polymerpartikel kontrolliert, mindestens ein weiteres Gen, das für ein bei der Bildung der Polymerpartikel beteiligtes Protein codiert, einbringt.
3. Verfahren nach Bedingung 2, wobei das mindestens eine weitere Gen, das für ein bei der Bildung der Polymerpartikel beteiligtes Protein codiert, für eine Thiolase, eine Reduktase oder eine Polymer-Synthase codiert.
4. Verfahren nach Bedingung 3, wobei das mindestens eine weitere Gen, das für ein bei der Bildung der Polymerpartikel beteiligtes Protein codiert, für die phaA-Thiolase, die phaB-Ketoacyl-Reduktase oder die phaC-Synthase aus *Ralstonia eutropha* codiert.
5. Verfahren nach einem der vorhergehenden Bedingung, wobei man das zumindest eine induzierbare Gen, das für ein Pratein codiert, das die Größe der Polymerpartikel kontrolliert, aus der Familie der Phasin-ähnlichen Proteine ableitet.
6. Verfahren nach Bedingung 5, wobei man das zumindest eine induzierbare Gen, das für ein Protein codiert, das die Größe der Polymerpartikel kontrolliert, aus der Gruppe auswählt, die das Phasin-Gen phaP aus *Ralstonia eutropha* und das Pliäsin-Gen phaF aus *Pseudomonas oleovorans* umfasst
7. Verfahren nach einem der vorhergehenden Bedingung , wobei man zumindest ein zusätzliches Gen, das für eine Thiolase und/oder eine Polymer-Synthase codiert, in die Zelle einbringt
8. Verfahren nach einem der vorhergehenden Bedingung , wobei man in das Kulturmedium als Substrat für die Bildung der Polymerpartikel mindestens eine Fettsäure mit funktionellen Seitengruppen und besonders Bevorzugt mindestens eine Hydroxyfettsäure und/oder mindestens eine Mercaptoiettsäure und/oder mindestens einer β-Aminofettsäure einbringt.
9. Verfahren nach einem der vorhergehenden Bedingung , wobei man das Substrat dem Kulturmedium in einer solchen Menge zugibt, dass es ausreichend bemessen ist, um die Kontrolle der Größe der Polymerpartikel zu gewährleisten.
10. Verfahren nach einem der vorergehenden Bedingung , wobei man den verwendeten Mikroorganismus aus der Gattung auswählt, die *Ralstonia, Alcaligenes, Pseudomonas* und *Halobiforma* umfasst.
11. Verfahren nach Bedingung 10, wobei man den verwendeten Mikroorganismus aus der Gruppe auswählt, die *Ralstonia eutropha, Alcaligenes latus, Escheriehia coli, Pseudomonas fragi, Pseudomonas putida, Pseudomonas oleovorans, Pseudomonas aeruginosa, Pseudomonas fluouescens,* und *Halobiforma haloterrestris* umfasst.
12. Ein Verfahren nach einem der vorhergehenden Bedingung, wobei man die kultivierten Mikroorganismen auf an sich bekannte Weise aufschließt und die Polymerpartikel anschließend von den Zellresten abtrennt.
13. Ein Verfahren nach Bedingung 12, wobei man eine sich auf der Oberfläche der Polymerpartikel befindliche Lipidschicht von den gemäß dem Verfahren aus Bedingung 12 erhaltenen Polymerpatikeln abtrennt und durch eine Lipidschicht anderer Zusammensetzung ersetzt.
14. Verfahren nach einem der vorhergehenden. Bedingung, wobei man die Partikelgröße durch das zumindest eine induzierbare Gen so steuert, dass die gebildeten Polymerpartikel einen Durchmesser von 10 nm bis 3 µm, bevorzugt einen Durchmesser von 10 nm bis 900 nm, und besonders bevorzugt einen Durchmesser von 10 nm bis 100 nm haben.
15. Verfahren nach Bedingung 1 oder 2, wobei das durch das in Stufe a) eingebrachte zumindest eine induzierbare Gen, das für ein Protein codiert, das die Größe der Polymerpartikel kontrolliert, eine Polymerpartikelbindedomäne und zumindest eine Bindedomäne umfasst, wobei die zumindest eine Bindedomäne eine biologisch aktive Substanz und/oder ein Kopplungsreagenz binden kann
16. Verfahren nach Bedingung 2, wobei das durch das in Stufe a) eingebrachte mindestens ein weitere Gen, das für ein bei der Bildung der Polymerpartikel beteiligtes Protein codiert, eine Polymerpartikelbindedomäne und zumindest eine Bindedomäne umfasst, wobei die zumindest eine Bindedomäne eine biologisch aktive Substanz und/oder ein Kopplungsreagenz binden kann.
17. Verfahren nach Bedingung. 15 oder 16, wobei die Polymerpartikelbindedomäne einen Teil eines auf der Oberfläche des Polymerpartikels gebundenen Proteins umfasst, wobei man das Protein aus der Gruppe auswählt, die eine Polymer-Depolymerase, einen Polymer Regulator, eine Polymer-Synthase und ein die Partikelgröße kontrollierendes Protein umfasst.
18. Verfahren nach Bedingung 15, 16 oder 17, wobei man die zumindest eine Bindedomäne, die eine biologisch aktive Substanz und/oder ein Kopplungsreagenz binden kann, aus der Gruppe auswählt, die Oligopeptide, Enzyme, Abzyme oder nicht-katalytische Proteine umfasst.
19. Verfahren zur *in vitro* Herstellung von biologisch abbaubaren Polymerpartikeln, das umfasst:
   a) Bereitstellen einer für die Polymerpartikelbildung geeigneten Lösung mit zumindest einem Substrat;
   b) Eindringen eines Proteins in die Lösung, das dazu geeignet ist, die Größe der Polymerpartikel zu kontrollieren; und
   c) Einbringen mindestens eines weiteren Proteins, das an der Bildung der Polymerpartikel beteiligt ist.
20. Verfahren nach Bedingung 19, wobei man der für die Polymerpartikelbildung geeigneten Lösung in Stufe a) mindestens eine Fettsäure und eine Acyl-CoA Oxidase für die Bildung der Polymerpartikel zugibt.
21. Verfahren nach einem der Bedingung 19 oder 20, wobei man in Stufe a) zumindest ein Substrat dei für die Polymerpartikelbildung geeigneten Lösung in einer solchen Menge zugibt, dass es ausreichend bemessen ist, um die Kontrolle der Größe der Polymerpartikel zu gewährleisten.
22. Verfahren nach Bedingung 19 bis 21, wobei man in Stufe b) ein die Größe der Polymerpartikel kontrollierendes Protein einbringt, das man aus der Familie der Phasin-ähnlichen Proteine ableitet.
23. Verfahren nach Bedingung, 22, wobei man in Stufe b) ein die Größe der Polymerpartikel kontrollierendes Protein einbringt, das man aus der Gruppe auswählt, die das Phasin aus *Ralstonia eutropha* und das Phasin aus *Pseudomonas oleovorans* umfasst.
24. Verfahren nach einem der Bedingung 19 bis 23, wobei man für das mindestens eine weitere Protein aus Stufe c), das an der Bildung der Polymerpartikel beteiligt ist, eine Polymer-Sythase einsetzt.
25. Verfahren nach Bedingung 24, wobei man für das mindestens eine weitere Protein aus Stufe c), das an der Bildung der Polymerpartikel beteiligt ist, eine Polymer-Synthase einsetzt, die man aus der Gruppe auswählt, welche die Polymer-Synthase aus *R eutropha, P. oleovorans, P. putida* und *P. aeruginosa* umfasst.
26. Verfaren nach Bedingung 26 oder 27, wobei man die Polymer-Synthase der Lösung in einer solchen Menge zugibt, die ausreichend ist, um die Kontrolle der Größe der Polymerpartikel zu gewährleisten.
27. Verfahren nach einem der Bedingung 21 bis 26, wobei man in Stufe a) der Lösung mindestens eine pharmazeutisch aktive Substanz zugibt.
28. Verfahren nach einem der Bedingung 21 bis 29, wobei man zur Kontrolle der Zusammensetzung der Lipidschicht auf der Oberfläche des Polymerpartikels mindestens ein amphiphiles Molekül aus der Gruppe der Phospholipide und Etherlipide zur Lösung aus Schritt a) zugibt
29. Verfahren nach einem der Bedingung 19 bis 28, wobei man mindestens eines der in Stufe b) und c) eingebrachten Proteine so auswählt, das es eine Polymerpartikelbindedomäne und zumindest eine Bindedomäne umfasst, wobei die zumindest eine Bindedomäne eine biologisch aktive Substanz und/oder ein Kopplungsreagenz binden kann
30. Verfahren nach Bedingung 29, wobei die Polymerpartikelbindedomäne ein Teil des auf der Oberfläche des Polymerpartikels gebundenen Proteins ist, wobei man das Protein aus der Gruppe auswählt, welche eine Polymer-Depolymerase, einen Polymer-Regulator, eine Polymer-Synthase und ein die Partikelgröße kontrollierendes Protein umfasst.
31. Verfahren nach Bedingung 29 oder 30, wobei man die zumindest eine Bindedomäne, die eine biologisch aktive Substanz und/oder ein Kopplungsreagenz binden kann, aus der Gruppe auswählt, die Oligopeptide, Enzyme, Abzyme oder nicht-katalytischen Proteine umfasst
32. Polymerpartikel mit definierter Größe, mit einer Obeiflächenschicht aus amphiphilen Molekülen, in die mindestens ein Protein, das aus der Gruppe ausgewählt wird, die eine Polymer-Depolymerase, einen Polymer-Regulator, eine Polymer-Synthase und ein die Partikelgröße beeinflussendes Protein umfasst, wobei das mindestens eine Protein eine Polymerpartikelbindedomäne und eine Bindedomäne, die eine biologisch aktive Substanz und/oder ein Kopplungsreagenz binden kann, umfasst
33. Polymerpartikel nach Bedingung 32, hergestellt nach einem der in den Bedingung 1 bis 33 beschriebenen Verfahren.
34. Verwendung der Polymerpartikel nach den Bedingung 32 oder 33 für die Herstellung eines Arzneimittels, eines Pestizids oder eines Herbizides.
35. Verwendung nach Bedingung 34, wobei das Arzneimittel für die Behandlung von Erkrankungen des zentralen Nervensystems geeignet ist.

## Patentansprüche

1. Polymerpartikel mit zumindest einer Polymer-Synthase,
wobei es sich bei der zumindest einen Polymer-Synthase um ein Fusionsprotein handelt, das ein biologisch aktives Protein umfaßt, oder
wobei es sich bei der zumindest einen Polymer-Synthase um ein Fusionsprotein handelt, das dazu in der Lage ist, eine biologisch aktive Substanz direkt oder über ein Kopplungsreagenz zu binden, oder
wobei es sich bei der zumindest einen Polymer-Synthase um ein modifiziertes Protein handelt, das dazu in der Lage ist, eine biologisch aktive Substanz direkt oder über ein Kopplungsreagenz zu binden.

2. Polymerpartikel nach Anspruch 1, weiterhin mit zumindest einem Protein, wobei man das Protein aus der Gruppe auswählt, welche eine Polymer-Depolymerase, einen Polymer-Regulator, eine Polymer-Synthase und ein die Partikelgröße kontrollierendes Protein umfaßt,
wobei es sich bei dem zumindest einen Protein um ein Fusionsprotein handelt, das ein biologisch aktives Protein umfaßt, oder
wobei es sich bei dem zumindest einen Protein um ein Fusionsprotein handelt, das dazu in der Lage ist, eine biologisch aktive Substanz direkt oder über ein Kopplungsreagenz zu binden, oder
wobei es sich bei der zumindest einen Polymer-Synthase um ein modifiziertes Protein handelt, das dazu in der Lage ist, eine biologisch aktive Substanz direkt oder über ein Kopplungsreagenz zu binden.

3. Polymerpartikel nach Anspruch 1, wobei die zumindest eine Polymer-Synthase eine Polymerpartikelbindedomäne und zumindest eine Bindedomäne umfaßt, oder Polymerpartikel nach Anspruch 2, wobei die zumindest eine Polymer-Synthase und das zumindest eine Protein jeweils eine Polymerpartikelbindedomäne und zumindest eine Bindedomäne umfassen, wobei die zumindest eine Bindedomäne dazu in der Lage ist, die biologisch aktive Substanz und/oder ein Kopplungsreagenz zu binden.

4. Polymerpartikel nach einem der Ansprüche 3, wobei man die zumindest eine Bindedomäne aus der Gruppe auswählt, die Oligopeptide, Enzyme, Abzyme, nicht-katalytische Proteine, FLAG-Epitope und zumindest einen Cysteinrest umfaßt.

5. Polymerpartikel nach einem der Ansprüche 3 oder 4, wobei die zumindest eine Bindedomäne erzeugt wird, indem man die zumindest eine Polymer-Synthase oder das zumindest eine Protein oder sowohl die zumindest eine Polymer-Synthase und das zumindest eine Protein mit einem Kopplungsreagenz chemisch modifiziert.

6. Polymerpartikel nach einem der Ansprüche 1 bis 5, wobei zumindest eine pharmazeutisch aktive Substanz und/oder ein Farbstoff in das Polymerpartikel eingearbeitet wird.

7. Verwendung eines Polymerpartikels nach einem der Ansprüche 1 bis 6 für die Herstellung eines Arzneimittels.

8. Verfahren zur Bindung einer biologisch aktiven Substanz, das umfaßt:
a) Bereitstellung eines oder mehrerer Polymerpartikel nach einem der Ansprüche 1 bis 6, und
b) In-Kontakt-Bringen des einen oder der mehreren Polymerpartikel mit einer Probe, die eine biologisch aktive Substanz umfaßt, sodaß das biologisch aktive Protein oder die zumindest eine Polymer-Synthase die biologisch aktive Substanz bindet.

9. Verfahren nach Anspruch 8, wobei die biologisch aktive Substanz eine pharmazeutisch aktive Substanz umfaßt.

10. Verfahren zur Herstellung von biologisch abbaubaren Polymerpartikeln, das umfaßt:
a) Bereitstellen einer Zelle, wobei zumindest ein Gen in die Zelle eingebracht worden ist, wobei das Gen für eine Polymer-Synthase codiert;
wobei es sich bei der zumindest einen Polymer-Synthase um ein Fusionsprotein handelt, das ein biologisch aktives Protein umfaßt, oder
wobei es sich bei der zumindest einen Polymer-Synthase um ein Fusionsprotein handelt, das dazu in der Lage ist, eine biologisch aktive Substanz direkt oder über ein Kopplungsreagenz zu binden, oder
wobei es sich bei der zumindest einen Polymer-Synthase um ein modifiziertes Protein handelt, das dazu in der Lage ist, eine biologisch aktive Substanz direkt oder über ein Kopplungsreagenz zu binden; und
b) Kultivieren der Zelle in einem Kulturmedium unter Bedingungen, die zur Herstellung der biologisch abbaubaren Polymerpartikel durch die Zelle geeignet sind.

11. Verfahren zur *in vitro* Herstellung von biologisch abbaubaren Polymerpartikeln, das umfaßt:
a) Bereitstellen einer für die Polymerpartikelbildung geeigneten Lösung mit zumindest einem Substrat;
b) Einbringen zumindest einer Polymer-Synthase in die Lösung,
wobei es sich bei der zumindest einen Polymer-Synthase um ein Fusionsprotein handelt, das ein biologisch aktives Protein umfaßt, oder
wobei es sich bei der zumindest einen Polymer-Synthase um ein Fusionsprotein handelt, das dazu in der Lage ist, eine biologisch aktive Substanz direkt oder über ein Kopplungsreagenz zu binden, oder
wobei es sich bei der zumindest einen Polymer-Synthase um ein modifiziertes Protein handelt, das dazu in der Lage ist, eine biologisch aktive Substanz direkt oder über ein Kopplungsreagenz zu binden.

12. Verfahren nach Anspruch 10 oder 11, wobei die zumindest eine Polymer-Synthase eine Polymerpartikelbindedomäne und zumindest eine Bindedomäne umfaßt, wobei die zumindest eine Bindedomäne dazu in der Lage ist, eine biologisch aktive Substanz und/oder über ein Kopplungsreagenz zu binden.

13. Verfahren nach Anspruch 12, wobei man die zumindest eine Bindedomäne, die dazu in der Lage ist, die biologisch aktive Substanz und/oder das Kopplungsreagenz zu binden, aus der Gruppe auswählt, die Oligopeptide, Enzyme, Abzyme, nicht-katalytische Proteine, FLAG-Epitope oder zumindest einen Cysteinrest umfaßt.

14. Partikel nach einem der Ansprüche 1 bis 7, Methode nach Anspruch 8 oder 9 oder Verfahren nach einem der Ansprüche 10 bis 13, wobei es sich bei dem biologisch aktiven Protein bzw. der biologisch aktiven Substanz um ein Oligopeptid, Enzym, Abzym, nichtkatalytisches Protein oder einen Antikörper handelt.

15. Partikel, Verwendung, Methode oder Verfahren nach einem der Ansprüche 1 bis 14, wobei das Partikel einen Durchmesser von 10 nm bis 3 µm, 10 nm bis 100 nm , oder 50 nm bis 500 nm aufweist.


Sequenzliste

<110> Rehm, PD Dr. Bern H.A.

<120> Verfahren zur Herstellung von biologisch abbaubaren,
funktionalisierten Polymerpartikeln und deren Verwendung als
Arzneimittelträger

<130> P200428

<150> DE 102 40 034.0
<151> 2002-08-30

<160> 15

<170> PatentIn version 3.2

<210> 1
<211> 29
<212> DNA
<213> Artificial

<220>
<223> Primer

<400> 1
aaaggcccca tggtcctcac cccggaaca 29

<210> 2
<211> 33
<212> DNA
<213> Artificial

<220>
<223> Primer

<400> 2
aaaggccgga tcctcagggc actaccttca tcg 33

<210> 3
<211> 708
<212> DNA
<213> Artificial

<220>
<223> Sequenz enthält phaP codierende DNA aus R. eutropha

<400> 3
aaaggcccca tgatcctcac cccggaacaa gttgcagcag cgcaaaaggc caacctcgaa 60

acgctgttcg gcctgaccac caaggcgttt gaaggcgtcg aaaagctcgt cgagctgaac 120

ctgcaggtcg tcaagacttc gttcgcagaa ggcgttgaca acgccaagaa ggcgctgtcg 180

gccaaggacg cacaggaact gctggccatc caggccgcag ccgtgcagcc ggttgccgaa 240

aagaccctgg cctacacccg ccacctgtat gaaatcgctt cggaaaccca gagcgagttc 300

accaaggtag ccgaggctca actggccgaa ggctcgaaga acgtgcaagc gctggtcgag 360

aacctcgcca agaacgcccc ggccggttcg gaatcgaccg tggccatcgt gaagtcggcg 420

atctccgctg ccaacaacgc ctacgagtcg gtgcagaagg cgaccaagca agcggtcgaa     480

atcgctgaaa ccaacttcca ggctgcggct acggctgcca ccaaggctgc ccagcaagcc     540

agcgccacgg cccgtacggc cacggcaaag aagacgacgg ctgcctgata actgcctgcg     600

ttgaagatgg accggctgcg gccggtccgt tggcaaagca tatcgacgcc tggcgtttgc     660

ggtgtgtttt gccaacgatg aaggtagtgc cctgaggatc cggccttt                  708

<210>  4
<211>  34
<212>  DNA
<213>  Artificial

<220>
<223>  Primer

<400>  4
aaagggccat ggctggcaag aagaattccg agaa                                  34

<210>  5
<211>  39
<212>  DNA
<213>  Artificial

<220>
<223>  Primer

<400>  5
aaagggggat cctcagatca gggtaccggt gcctgtctg                             39

<210>  6
<211>  793
<212>  DNA
<213>  Artificial

<220>
<223>  Sequenz enthält phaF codierende DNA aus P. oleovorans

<400>  6
aaagggccat ggctggcaag aagaattccg agaaagaagg cagctcctgg gtcggcggga      60

tcgagaagta ctcccgcaag atctggctgg cggggcttgg tatctattcg aagatcgacc     120

aggacggccc gaagctgttc gactcgctgg taaaggatgg cgagaaggcc gagaaacagg     180

cgaagaagac cgcagaagat gttgctgaaa ctgccaagtc gtcgaccact tcgcgtgtgt     240

cgggcgtgaa ggaccgtgcg ctaggcaagt ggagcgaact cgaagaggcc ttcgacaagc     300

gcctgaacag tgccatctcg cgccttggcg tgccgagccg caacgagatc aaggccctgc     360

accagcaggt ggacagcctg accaagcaga tcgagaaact caccggcgct tcggttaccc     420

cgatttcgtc gcgcactgca gccaaaccgg ctgcgagcaa ggcggcggcc aagccactgg     480

ccaagacggc agcggccaag cctgcggcaa aaaccgcggc agccaagccg gcagccaagg     540

ccgcagcggc taaacctgct gccaagactg cggcggccaa gcctgcggcg aaaccggcag     600

cggccaaacc ggctgtggcg aagaagcctg cagtgaagaa agcaccggcc aagccggcag 660

ccgccaagcc ggcagctcca gcggccagcg ccgctccggc cgctagcgca gttcggcgcc 720

cactgcggct ccggccagca acccgccttc ggcacagaca ggcaccggta ccctgatctg 780

aggatccccc ttt 793

<210> 7
<211> 54
<212> DNA
<213> Artificial

<220>
<223> Primer

<400> 7
gggctctaga aataaggaga tatacatatg tgttgtaaga acaataacga gctt 54

<210> 8
<211> 29
<212> DNA
<213> Artificial

<220>
<223> Primer

<400> 8
aaacgcggat ccttttcatc gttcatgca 29

<210> 9
<211> 1722
<212> DNA
<213> Artificial

<220>
<223> Sequenz enthält die für PhaC1 codierende DNA aus P. aeruginosa

<400> 9
gggctctaga aataaggaga tatacatatg tgttgtaaga acaataacga gcttcccaag 60

caagccgcgg aaaacacgct gaacctgaat ccggtgatcg gcatccgggg caaggacctg 120

ctcacctccg cgcgcatggt cctgctccag gcggtgcgcc agccgctgca cagcgccagg 180

cacgtggcgc atttcagcct ggagctgaag aacgtcctgc tcggccagtc ggagctacgc 240

ccaggcgatg acgaccgacg cttttccgat ccggcctgga gccagaatcc actgtacaag 300

cgctacatgc agacctacct ggcctggcgc aaggagctgc acagctggat cagccacagc 360

gacctgtcgc cgcaggacat cagtcgtggc cagttcgtca tcaacctgct gaccgaggcg 420

atgtcgccga ccaacagcct gagcaacccg gcggcggtca agcgcttctt cgagaccggc 480

ggcaagagcc tgctggacgg cctcggccac ctggccaagg acctggtgaa caacggcggg 540

atgccgagcc aggtggacat ggacgccttc gaggtgggca agaacctggc caccaccgag 600

ggcgccgtgg tgttccgcaa cgacgtgctg gaactgatcc agtaccggcc gatcaccgag 660

tcggtgcacg aacgcccgct gctggtggtg ccgccgcaga tcaacaagtt ctacgtcttc 720

gacctgtcgc cggacaagag cctggcgcgc ttctgcctgc gcaacggcgt gcagaccttc 780

atcgtcagtt ggcgcaaccc gaccaagtcg cagcgcgaat ggggcctgac cacctatatc 840

gaggcgctca aggaggccat cgaggtagtc ctgtcgatca ccggcagcaa ggacctcaac 900

ctcctcggcg cctgctccgg cgggatcacc accgcgaccc tggtcggcca ctacgtggcc 960

agcggcgaga agaaggtcaa cgccttcacc caactggtca gcgtgctcga cttcgaactg 1020

aatacccagg tcgcgctgtt cgccgacgag aagactctgg aggccgccaa gcgtcgttcc 1080

taccagtccg gcgtgctgga gggcaaggac atggccaagg tgttcgcctg gatgcgcccc 1140

aacgacctga tctggaacta ctgggtcaac aactacctgc tcggcaacca gccgccggcg 1200

ttcgacatcc tctactggaa caacgacacc acgcgcctgc ccgccgcgct gcacggcgag 1260

ttcgtcgaac tgttcaagag caacccgctg aaccgccccg gcgccctgga ggtctccggc 1320

acgcccatcg acctgaagca ggtgacttgc gacttctact gtgtcgccgg tctgaacgac 1380

cacatcaccc cctgggagtc gtgctacaag tcggccaggc tgctgggtgg caagtgcgag 1440

ttcatcctct ccaacagcgg tcacatccag agcatcctca acccaccggg caaccccaag 1500

gcacgcttca tgaccaatcc ggaactgccc gccgagccca aggcctggct ggaacaggcc 1560

ggcaagcacg ccgactcgtg gtggttgcac tggcagcaat ggctggccga acgctccggc 1620

aagacccgca aggcgcccgc cagcctgggc aacaagacct atccggccgg cgaagccgcg 1680

cccggaacct acgtgcatga acgatgaaaa ggatccgcgt tt 1722

<210> 10
<211> 36
<212> DNA
<213> Artificial

<220>
<223> Primer

<400> 10
tatgactagt gattataaag atgatgatga taaaca 36

<210> 11
<211> 36
<212> DNA
<213> Artificial

<220>
<223> Primer

<400> 11
tatgtttatc atcatcatct ttataatcac tagtca 36

<210> 12
<211> 1716

<212>  DNA
<213>  Artificial

<220>
<223>  Sequenz enthält die für PhaC1 codierende DNA aus P. aeruginosa
       und die für ein FLAG-Epitop codierende DNA

<400>  12
atgactagtg attataaaga tgatgatgat aaacatatga gtcagaagaa caataacgag      60

cttcccaagc aagccgcgga aaacacgctg aacctgaatc cggtgatcgg catccggggc     120

aaggacctgc tcacctccgc gcgcatggtc ctgctccagg cggtgcgcca gccgctgcac     180

agcgccaggc acgtggcgca tttcagcctg gagctgaaga acgtcctgct cggccagtcg     240

gagctacgcc caggcgatga cgaccgacgc ttttccgatc cggcctggag ccagaatcca     300

ctgtacaagc gctacatgca gacctacctg gcctggcgca aggagctgca cagctggatc     360

agccacagcg acctgtcgcc gcaggacatc agtcgtggcc agttcgtcat caacctgctg     420

accgaggcga tgtcgccgac caacagcctg agcaacccgg cggcggtcaa gcgcttcttc     480

gagaccggcg gcaagagcct gctggacggc ctcggccacc tggccaagga cctggtgaac     540

aacggcggga tgccgagcca ggtggacatg gacgccttcg aggtgggcaa gaacctggcc     600

accaccgagg gcgccgtggt gttccgcaac gacgtgctgg aactgatcca gtaccggccg     660

atcaccgagt cggtgcacga acgcccgctg ctggtggtgc cgccgcagat caacaagttc     720

tacgtcttcg acctgtcgcc ggacaagagc ctggcgcgct tctgcctgcg caacggcgtg     780

cagaccttca tcgtcagttg gcgcaacccg accaagtcgc agcgcgaatg gggcctgacc     840

acctatatcg aggcgctcaa ggaggccatc gaggtagtcc tgtcgatcac cggcagcaag     900

gacctcaacc tcctcggcgc ctgctccggc gggatcacca ccgcgaccct ggtcggccac     960

tacgtggcca gcggcgagaa gaaggtcaac gccttcaccc aactggtcag cgtgctcgac    1020

ttcgaactga atacccaggt cgcgctgttc gccgacgaga agactctgga ggccgccaag    1080

cgtcgttcct accagtccgg cgtgctggag ggcaaggaca tggccaaggt gttcgcctgg    1140

atgcgcccca acgacctgat ctggaactac tgggtcaaca actacctgct cggcaaccag    1200

ccgccggcgt tcgacatcct ctactggaac aacgacacca cgcgcctgcc cgccgcgctg    1260

cacggcgagt tcgtcgaact gttcaagagc aacccgctga accgccccgg cgccctggag    1320

gtctccggca cgcccatcga cctgaagcag gtgacttgcg acttctactg tgtcgccggt    1380

ctgaacgacc acatcacccc ctgggagtcg tgctacaagt cggccaggct gctgggtggc    1440

aagtgcgagt tcatcctctc caacagcggt cacatccaga gcatcctcaa cccaccgggc    1500

aaccccaagg cacgcttcat gaccaatccg gaactgcccg ccgagcccaa ggcctggctg    1560

gaacaggccg gcaagcacgc cgactcgtgg tggttgcact ggcagcaatg gctggccgaa    1620

cgctccggca agacccgcaa ggcgcccgcc agcctgggca acaagaccta tccggccggc    1680

gaagccgcgc ccggaaccta cgtgcatgaa cgatga                                1716

<210>  13
<211>  34
<212>  DNA
<213>  Artificial

<220>
<223>  Primer

<400>  13
ggactagtat gaccatgatt acggattcac tggc                                  34

<210>  14
<211>  41
<212>  DNA
<213>  Artificial

<220>
<223>  Primer

<400>  14
ccactagttt tttgacacca gaccaactgg taatggtagc g                          41

<210>  15
<211>  3088
<212>  DNA
<213>  Artificial

<220>
<223>  Sequenz enthält das lacZ Gen aus E. coli

<400>  15
ggactagtat gaccatgatt acggattcac tggccgtcgt tttacaacgt cgtgactggg      60

aaaaccctgg cgttacccaa cttaatcgcc ttgcagcaca tccccctttc gccagctggc     120

gtaatagcga agaggcccgc accgatcgcc cttcccaaca gttgcgcagc ctgaatggcg     180

aatggcgctt tgcctggttt ccggcaccag aagcggtgcc ggaaagctgg ctggagtgcg     240

atcttcctga ggccgatact gtcgtcgtcc cctcaaactg gcagatgcac ggttacgatg     300

cgcccatcta caccaacgtg acctatccca ttacggtcaa tccgccgttt gttcccacgg     360

agaatccgac gggttgttac tcgctcacat ttaatgttga tgaaagctgg ctacaggaag     420

gccagacgcg aattattttt gatggcgtta actcggcgtt tcatctgtgg tgcaacgggc     480

gctgggtcgg ttacggccag gacagtcgtt tgccgtctga atttgacctg agcgcatttt     540

tacgcgccgg agaaaaccgc ctcgcggtga tggtgctgcg ctggagtgac ggcagttatc     600

tggaagatca ggatatgtgg cggatgagcg gcattttccg tgacgtctcg ttgctgcata     660

aaccgactac acaaatcagc gatttccatg ttgccactcg ctttaatgat gatttcagcc     720

gcgctgtact ggaggctgaa gttcagatgt gcggcgagtt gcgtgactac ctacgggtaa     780

cagtttcttt atggcagggt gaaacgcagg tcgccagcgg caccgcgcct ttcggcggtg     840

aaattatcga tgagcgtggt ggttatgccg atcgcgtcac actacgtctg aacgtcgaaa 900
acccgaaact gtggagcgcc gaaatcccga atctctatcg tgcggtggtt gaactgcaca 960
ccgccgacgg cacgctgatt gaagcagaag cctgcgatgt cggtttccgc gaggtgcgga 1020
ttgaaaatgg tctgctgctg ctgaacggca agccgttgct gattcgaggc gttaaccgtc 1080
acgagcatca tcctctgcat ggtcaggtca tggatgagca gacgatggtg caggatatcc 1140
tgctgatgaa gcagaacaac tttaacgccg tgcgctgttc gcattatccg aaccatccgc 1200
tgtggtacac gctgtgcgac cgctacggcc tgtatgtggt ggatgaagcc aatattgaaa 1260
cccacggcat ggtgccaatg aatcgtctga ccgatgatcc gcgctggcta ccggcgatga 1320
gcgaacgcgt aacgcgaatg gtgcagcgcg atcgtaatca cccgagtgtg atcatctggt 1380
cgctggggaa tgaatcaggc cacggcgcta atcacgacgc gctgtatcgc tggatcaaat 1440
ctgtcgatcc ttcccgcccg gtgcagtatg aaggcggcgg agccgacacc acggccaccg 1500
atattatttg cccgatgtac gcgcgcgtgg atgaagacca gcccttcccg gctgtgccga 1560
aatggtccat caaaaaatgg ctttcgctac ctggagagac gcgcccgctg atcctttgcg 1620
aatacgccca cgcgatgggt aacagtcttg gcggtttcgc taaatactgg caggcgtttc 1680
gtcagtatcc ccgtttacag ggcggcttcg tctgggactg ggtggatcag tcgctgatta 1740
aatatgatga aaacggcaac ccgtggtcgg cttacggcgg tgattttggc gatacgccga 1800
acgatcgcca gttctgtatg aacggtctgg tctttgccga ccgcacgccg catccagcgc 1860
tgacggaagc aaaacaccag cagcagtttt tccagttccg tttatccggg caaaccatcg 1920
aagtgaccag cgaatacctg ttccgtcata gcgataacga gctcctgcac tggatggtgg 1980
cgctggatgg taagccgctg gcaagcggtg aagtgcctct ggatgtcgct ccacaaggta 2040
aacagttgat tgaactgcct gaactaccgc agccggagag cgccgggcaa ctctggctca 2100
cagtacgcgt agtgcaaccg aacgcgaccg catggtcaga agccgggcac atcagcgcct 2160
ggcagcagtg gcgtctggcg gaaaacctca gtgtgacgct ccccgccgcg tcccacgcca 2220
tcccgcatct gaccaccagc gaaatggatt tttgcatcga gctgggtaat aagcgttggc 2280
aatttaaccg ccagtcaggc tttctttcac agatgtggat tggcgataaa aaacaactgc 2340
tgacgccgct gcgcgatcag ttcacccgtg caccgctgga taacgacatt ggcgtaagtg 2400
aagcgacccg cattgaccct aacgcctggg tcgaacgctg gaaggcggcg ggccattacc 2460
aggccgaagc agcgttgttg cagtgcacgg cagatacact tgctgatgcg gtgctgatta 2520
cgaccgctca cgcgtggcag catcagggga aaaccttatt tatcagccgg aaaacctacc 2580
ggattgatgg tagtggtcaa atggcgatta ccgttgatgt tgaagtggcg agcgatacac 2640
cgcatccggc gcggattggc ctgaactgcc agctggcgca ggtagcagag cgggtaaact 2700
ggctcggatt agggccgcaa gaaaactatc ccgaccgcct tactgccgcc tgttttgacc 2760

gctgggatct gccattgtca gacatgtata ccccgtacgt cttcccgagc gaaaacggtc 2820
tgcgctgcgg gacgcgcgaa ttgaattatg gcccacacca gtggcgcggc gacttccagt 2880
tcaacatcag ccgctacagt caacagcaac tgatggaaac cagccatcgc catctgctgc 2940
acgcggaaga aggcacatgg ctgaatatcg acggtttcca tatggggatt ggtggcgacg 3000
actcctggag cccgtcagta tcggcggaat tccagctgag cgccggtcgc taccattacc 3060
agttggtctg gtgtcaaaaa actagtgg 3088